# FASCICULE DE BREVET EUROPEEN

(11) **EP 1 095 940 B1**
(45) Date de publication et mention de la délivrance du brevet: **21.07.2004**
(21) Numéro de dépôt: 00402985.6
(22) Date de dépôt: 27.10.2000
(51) Int. Cl.: C07D 519/00, C07D 493/04, C07H 17/04, A61K 31/365, A61K 31/36, A61K 31/70, A61P 35/00

(54) **Dérivés de 9-(3,5-diméthoxyphényl)-5,8,8a,9-tétrahydrofuro[3',4':6,7]naphto[2,3-d][1,3]dioxol-6(5aH)-one, leur procédé de préparation et les compositions pharmaceutiques qui les contiennent**
9-(3,5-dimethoxyphenyl)-5,8,8a,9-tetrahydrofuro[3',4':6,7]naphto[2,3-d][1,3]dioxol-6(5aH)-on - Derivate, Verfahren zu ihrer Herstellung und diese enthaltende pharmazeutische Zubereitungen
Derivatives of 9-(3,5-dimethoxyphenyl)-5,8,8a,9-tetrahydrofuro[3',4':6,7]naphto[2,3-d][1,3]dioxol-6(5aH)-one, method for their preparation and pharmaceutical compositions containing them

(30) Priorité: 28.10.1999 FR 9913514
(43) Date de publication de la demande: 02.05.2001
(73) Titulaire: LES LABORATOIRES SERVIER, 92200 Neuilly sur Seine (FR)
(72) Inventeur: Monneret, Claude, 75012 Paris (FR); Bertounesque, Emmanuel, 75001 Paris (FR); Meresse, Philippe, 59218 Salesches (FR); Atassi, Ghanem, 92210 Saint-Cloud (FR); Pierre, Alain, 78580 Les Alluets le Roi (FR); Pfeiffer, Bruno, 95320 Saint Leu la Foret (FR); Renard, Pierre, 78150 Le Chesnay (FR)

(56) Documents cités:
- EP-A- 0 196 618
- EP-A- 0 415 453
- WO-A-97/13776
- US-A- 3 634 459
- BEARD, ANDREW R. ET AL: "Synthesis of analogs of 4-deoxypodophyllotoxin" TETRAHEDRON (1987), 43(18), 4207-15, XP002146181
- KUHNT, MICHAELA ET AL: "Lignans and other compounds from the Mixe Indian medicinal plant Hyptis verticillata" PHYTOCHEMISTRY (1994), 36(2), 485-9, XP000939013
- KUO, YUEH HSIUNG ET AL: "A new lignan, formosalactone, from the bark of Juniperus formosana hay. var. concolor hay" HETEROCYCLES (1993), 36(3), 529-35, XP002146180
- CHOY, WILLIAM: "An enantioselective total synthesis of epiisopodophyllotoxin" TETRAHEDRON (1990), 46(7), 2281-6, XP002146182

## Description

La présente invention concerne de nouveaux dérivés de 9-(3,5-diméthoxyphényl)-5,8,8a,9-tétrahydrofuro[3',4':6,7]naphto[2,3-*d*][1,3]dioxol-6(5a*H*)-one, leur procédé de préparation et les compositions pharmaceutiques qui les contiennent.

Les composés de invention constituent des dérivés de la podophyllotoxine, un lignane naturel connu pour son utilité dans le traitement du cancer. D'autres dérivés synthétiques tels que l'étoposide et la téniposide sont utilisés de façon courante comme agent chimiothérapeutique pour le traitement notamment du cancer du poumon à petites cellules. Ces différents composés agissent en inhibant l'activité catalytique de la topoisomérase II.

Diverses modifications ont été réalisées sur ces dérivés, comme celles décrites dans les demandes de brevet JP 948782, WO 97/13776 et US 3,634,459. Néanmoins, les besoins de la thérapeutique anticancéreuse exigent le développement constant de nouveaux agents antitumoraux et cytotoxiques, dans le but d'obtenir des médicaments à la fois plus actifs, plus solubles et mieux tolérés.

Y. H. KUO et al. décrivent dans Heterocycles, 36 (1993), 529-535, un isomère de la 9-(3,4,5-triméthoxyphényl)-5,8,8a,9-tétrahydrofuro[3',4':6,7]naphto[2,3-*d*][1,3]dioxol-6(5a*H*-one (formosalactone), extrait de l'écorce de *Juniperus formosana.*

Les composés de la présente invention, outre le fait qu'ils soient nouveaux, présentent une activité in-vivo et in-vitro surprenante et supérieure à celle observée jusqu'ici. Ainsi, les composés découverts par la Demanderesse possèdent des propriétés qui les rendent particulièrement utiles pour le traitement des cancers.

Plus particulièrement, la présente invention concerne les composés de formule (I) : 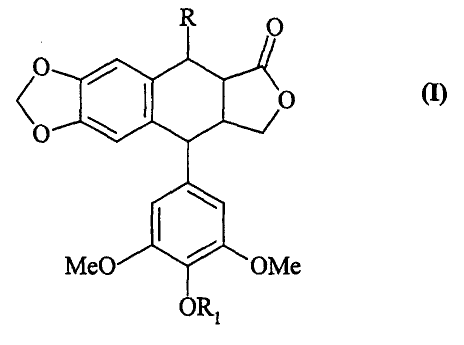 dans laquelle :
R représente :
   - *un groupement de formule (i) :* 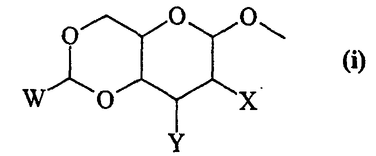 dans laquelle :
      - X et Y,: identiques ou différents, représentent un groupement choisi parmi hydrogène, hydroxy, alkoxy (C₁-C₆) linéaire ou ramifié, amino, alkylamino (C₁-C₆) linéaire ou ramifié, ou dialkylamino (C₁-C₆) linéaire ou ramifié,
      - W: représente un atome d'hydrogène, un groupement alkyle (C₁-C₆) linéaire ou ramifié, aryle ou hétéroaryle,
   - *ou un groupement de formule (ii) :*

      - A - G **(ii)**

      dans laquelle :
      - A: représente une liaison simple ou une chaîne alkylène (C₁-C₆) linéaire ou ramifiée, éventuellement substituée par un ou plusieurs groupements, identiques ou différents, choisis parmi halogène et hydroxy, et comportant éventuellement une insaturation,
      - G: représente un groupement choisi parmi atome d'hydrogène, cycloalkyle, hétérocycloalkyle, aryle, hétéroaryle, -OR₂, -O-T₁-NR₃R₄, -O-T₁-NR₂-T'₁-NR₃R₄, NR₃R₄, -NR₂-T₁-NR₃R₄, -NR₂-T₁-OR₅, -NR₂-T₁-CO₂R₆, NR₂T₁-C(O)R₆, -C(O)-NR₃R₄, -C(O)-NR₂-T₂, -O-C(O)T₂, -O-C(S)-T₂, -NR₂-C(O)-T₂, NR₂-C(S)T₂, -O-C(O)-O-T₂, -O-C(O)-NR₂-T₂, -O-C(S)-O-T₂, -O-C(S)-NR₂-T₂, -NR₂-C(O)-O-T₂, -NR₂-C(O)-NR₆-T₂, -NR₂-C(S)-O-T₂, -NR₂-C(S)-NR₆-T₂, et -NR₂-SO₂-T₃ dans lesquels :
      R₂ représente un atome d'hydrogène, un groupement alkyle (C₁-C₆) linéaire ou ramifié, aryle ou arylalkyle (C₁-C₆) linéaire ou ramifié,
      T₁, T'₁, identiques ou différents, représentent une chaîne alkylène (C₁-C₆) linéaire ou ramifié,
      R₃, R₄, identiques ou différents, indépendamment l'un de l'autre, représentent :
      - un atome d'hydrogène, un groupement alkyle (C₁-C₆) linéaire ou ramifié, (éventuellement substitué par un ou plusieurs groupements hydroxy), aryle, arylalkyle (C₁-C₆) linéaire ou ramifié, cycloakyle, hétéroarylalkyle (C₁-C₆) linéaire ou ramifié, hétéroaryle, hétérocycloalkyle, hétérocycloalkylalkyle (C₁-C₆) linéaire ou ramifié,
      - ou forment ensemble avec l'atome d'azote qui les porte un hétérocycle de 5 à 7 chaînons, saturé ou insaturé, monocyclique, et comportant éventuellement un second hétéroatome choisi parmi oxygène et azote, ledit hétérocycle étant éventuellement substitué, par un ou plusieurs groupements choisis parmi halogène, hydroxy, alkyle (C₁-C₆) linéaire ou ramifié, et hétérocycle,

      R₅ représente un groupement alkyle (C₁-C₆) linéaire ou ramifié, aryle, ou arylalkyle (C₁-C₆) linéaire ou ramifié,
      R₆ représente un atome d'hydrogène, un groupement alkyle (C₁-C₆) linéaire ou ramifié, aryle, ou arylalkyle (C₁-C₆) linéaire ou ramifié,
      T₂ représente un groupement choisi parmi atome d'hydrogène, alkyle (C₁-C₆) linéaire ou ramifié (éventuellement substitué par un ou plusieurs atomes d'halogène), aryle, arylalkyle (C₁-C₆) linéaire ou ramifié, cycloalkyle, cycloalkylalkyle (C₁-C₆) linéaire ou ramifié, hétérocycloalkyle et hétérocycloalkylalkyle (C₁-C₆) linéaire ou ramifié, ou T₂ représente une chaîne alkylène (C₁-C₆) linéaire ou ramifié, ladite chaîne étant substituée par un ou plusieurs groupements, identiques ou différents, choisis parmi -NR₃R₄, -OR₂, -CO₂R₆, -NR₂-C(O)R₆, -NR₂-CO₂R₆, -C(O)R₆, -C(O)NR₃R₄, -NR₂-T₁-NR₃R₄, -NR₂-Ti-OR₆, et -O-T₁-NR₃R₄ dans lesquels R₂, R₃, R₄, R₆ et T₁ sont tels que définis précédemment,
      T₃ représente un groupement choisi parmi alkyle (C₁-C₂₀) linéaire ou ramifié (éventuellement substitué par un ou plusieurs groupements choisis parmi halogène, -OR₂, -NR₂R₆, nitro, cyano, azide), aryle, arylalkyle (C₁-C₆) linéaire ou ramifié, cycloalkyle, cycloalkylalkyle (C₁-C₆) linéaire ou ramifié, hétérocycloalkyle, hétérocycloalkylalkyle (C₁-C₆) linéaire ou ramifié, hétéroaryle et hétéroarylalkyle (C₁-C₆) linéaire ou ramifié,
R₁représente un groupement choisi parmi hydrogène, alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, alkylcarbonyle (C₁-C₆) linéaire ou ramifié, arylcarbonyle, arylalkylcarbonyle (C₁-C₆) linéaire ou ramifié, hétérocycloalkylcarbonyle, alkylsulfonyle (C₁-C₆) linéaire ou ramifié, arylsulfonyle, arylalkylsulfonyle (C₁-C₆) linéaire ou ramifié, phosphonique, alkoxycarbonyle (C₁-C₆) linéaire ou ramifié, aryloxycarbonyle, et arylalkoxy(C₁-C₆)carbonyle linéaire ou ramifié,
leurs isomères ainsi que leurs sels d'addition à un acide ou à une base pharmaceutiquement acceptable,
étant entendu que :
* *par aryle,* on comprend un groupement phényle, biphényle, naphtyle, dihydronaphtyle, tétrahydronaphtyle, indényle ou indanyle, chacun de ces groupements comportant éventuellement une ou plusieurs substitutions, identiques ou différentes, choisies parmi halogène, hydroxy, alkyle (C₁-C₆) linéaire ou ramifié, alkoxy (C₁-C₆) linéaire ou ramifié, cyano, nitro, amino, alkylamino (C₁-C₆) linéaire ou ramifié, dialkylamino (C₁-C₆) linéaire ou ramifié, alkylsulfonyle (C₁-C₆) linéaire ou ramifié, alkylsulfonylamino (C₁-C₆) linéaire ou ramifié, carboxy, alkoxycarbonyle (C₁-C₆) linéaire ou ramifié, aryloxycarbonyle, arylalkoxycarbonyle (C₁-C₆) linéaire ou ramifié, hydroxyalkyle (C₁-C₆) linéaire ou ramifié, trihalogénoalkyle (C₁-C₆) linéaire ou ramifié, méthylènedioxy, éthylènedioxy, morpholinyle, pipéridyle, pipérazinyle, alkylcarbonyloxy (C₁-C₆) linéaire ou ramifié, et alkylcarbonyle (C₁-C₆) linéaire ou ramifié,
* *par hétéroaryle,* on comprend un monocycle aromatique, un bicycle aromatique, ou un bicycle dont l'un des cycles est aromatique et l'autre cycle est partiellement hydrogéné, de 5 à 12 chaînons, comportant au sein du système cyclique un, deux ou trois hétéroatomes, identiques ou différents, choisis parmi oxygène, azote et soufre, ledit hétéroaryle pouvant éventuellement être substitué par les mêmes substitutions que celles décrites dans le cas du groupement aryle,
* *par cycloalkyle,* on comprend un groupement monocyclique ou bicyclique, saturé ou insaturé mais sans caractère aromatique, comportant de 3 à 10 atomes de carbone, étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, hydroxyalkyle (C₁-C₆) linéaire ou ramifié, amino, alkylamino (C₁-C₆) linéaire ou ramifié, dialkylamino (C₁-C₆) linéaire ou ramifié, pipéridyle, pipérazinyle, et morpholinyle,
* *par hétérocycloalkyle,* on comprend un cycloalkyle tel que défini précédemment, comportant un ou deux hétéroatomes, identiques ou différents, choisis parmi oxygène, azote et soufre, ledit hétérocycloalkyle étant éventuellement substitué par un ou plusieurs substituants tels que ceux décrits dans le cas du groupement cycloalkyle.
étant entendu également que R₁ ne représente pas un groupement méthyle lorsque R représente un groupement -A-G dans lequel A représente une liaison simple et G représente un atome d'hydrogène.

Parmi les acides pharmaceutiquement acceptables, on peut citer à titre non limitatif, les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les substituants R₁ préférés selon l'invention sont l'atome d'hydrogène, et les groupements alkyle (C₁-C₆) linéaire ou ramifié, alkoxycarbonyle (C₁-C₆) linéaire ou ramifié, aryloxycarbonyle et arylalkoxycarbonyle (C₁-C₆) linéaire ou ramifié.

D'une façon très avantageuse, les composés préférés de l'invention sont ceux pour lesquels R₁ représente un atome d'hydrogène.

Selon une variante avantageuse de l'invention, les composés préférés de l'invention sont ceux pour lesquels R représente un groupement de formule (i) dans laquelle :
◆ X représente un groupement amino, alkylamino (C₁-C₆) linéaire ou ramifié ou dialkylamino (C₁-C₆) linéaire ou ramifié et Y représente un groupement hydroxy, ou X et Y, identiques, représentent chacun un groupement hydroxy, ou X représente un atome d'hydrogène et Y représente alors un groupement amino, alkylamino (C₁-C₆) linéaire ou ramifié, ou dialkylamino (C₁-C₆) linéaire ou ramifié, et
◆ W représente un groupement méthyle ou 2-thiényle.

D'une façon préférentielle, les composés préférés de l'invention sont ceux pour lesquels R représente un groupement de formule (i) telle que : 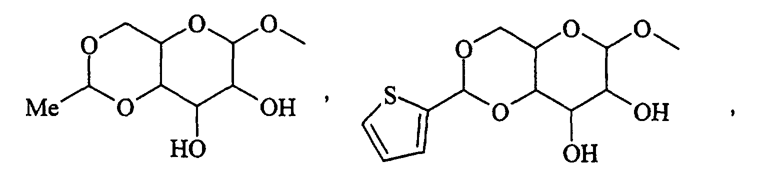 et

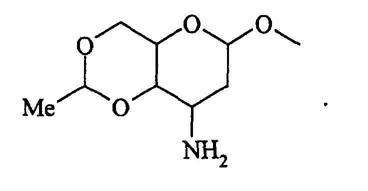

Selon une autre variante particulièrement avantageuse de l'invention, les composés préférés sont ceux pour lesquels R représente un groupement de formule (ii) dans laquelle :
◆ A représente une liaison simple, et
◆ G représente un groupement choisi parmi O-T₁-NR₃R₄, -O-T₁-NR₂-T₁-NR₃R₄, - NR₂-T₁-NR₃R₄, NR₃R₄, -NR₂-T₁-OR₅, -O-C(O)-NR₂-T₂ et -NR₂-SO₂-T₃ dans lesquels T₁, T'₁, T₂, T₃, R₂, R₃, R₄ et R₅ sont tels que définis dans la formule (I).

D'une façon particulièrement préférentielle, les composés préférés de invention sont ceux dans lesquels R représente un groupement de formule (ii) dans laquelle :
◆ A représente une liaison simple, et
◆ G représente un groupement -O-C(O)-NR₂-T₂ dans lequel R₂ et T₂ sont tels que définis dans la formule (I), et de façon avantageuse, R₂ représente un atome d'hydrogène ou un groupement alkyle (C₁-C₆) linéaire ou ramifié et T₂ représente un groupement aryle éventuellement substitué ou une chaîne alkylène (C₁-C₆) linéaire ou ramifiée substituée par un groupement -NR₃R₄ dans lequel R₃ et R₄ sont tels que définis dans la formule (I).

D'après une troisième variante avantageuse, les composés préférés de l'invention sont ceux pour lesquels R représente un groupement de formule (ii) dans laquelle :
◆ A représente une liaison simple, et
◆ G représente un groupement -NR₂-SO₂-T₃ dans lequel R₂ et T₃ sont tels que définis dans la formule (I), et de façon avantageuse, R₂ représente un atome d'hydrogène ou un groupement alkyle (C₁-C₆) linéaire ou ramifié et T₃ représente un groupement alkyle (C₁-C₆) linéaire ou ramifié, aryle ou hétéroaryle.

Les composés préférés de l'invention sont :
- le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 4-fluorophénylcarbamate,
- le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 2-(diméthylamino)éthyl(méthyl)carbamate,
- le 9-(4-hydroxy-3,5-diméthoxyphényl)-6-oxo-5,5a,6,8,8a,9-hexahydrofuro[3',4':6,7] naphto[2,3-*d*][1,3]-dioxol-5-yl 2-(diméthylamino)éthylcarbamate,
- le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 3-(diméthylamino)propyl(méthyl)carbamate,
- et le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 3-(diméthylamino)propylcarbamate.

Les isomères ainsi que les sels d'addition à un acide ou à une base pharmaceutiquement acceptable des composés préférés font partie intégrante de l'invention.

Ainsi, les isomères respectifs (5*R*,5a*R*,8a*S*,9*R*), (5*R*,5a*S*,8a*R*,9*R*), (5*R*,5a*R*,8a*S*,9*R*), (5*R*,5a*R*,8a*S*,9*R*), et (5*R*,5a*S*,8a*R*,9*R*), de chacun des cinq composés de formule (I) décrits spécifiquement précédemment, appartiennent à l'invention.

La présente invention s'étend également au procédé de préparation des composés de formule (I), caractérisé en ce que soit utilisé comme produit de départ un composé de formule (II) : 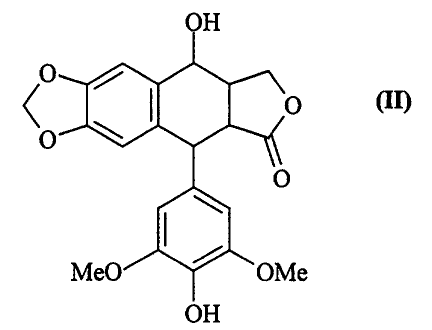 composé de formule (II) dont les fonctions hydroxy sont protégées par un groupement protecteur silylé classiquement utilisé en chimie organique, selon des conditions opératoires usuelles de la synthèse organique, pour conduire au composé de formule (III) : 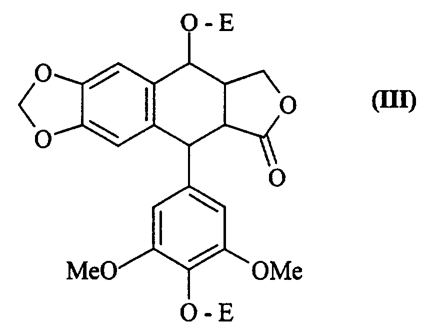 dans laquelle E représente un groupement protecteur silylé,
composé de formule (III) qui est traité par un agent de réduction pour conduire au composé de formule (IV) : 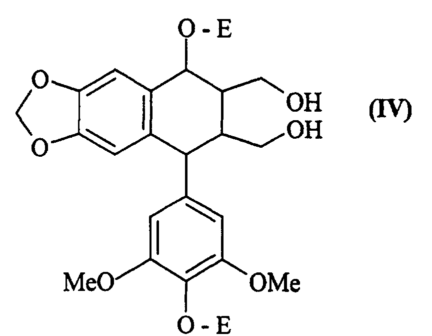 dans laquelle E est tel que défini précédemment,
composé de formule (IV) qui est mis à réagir, en milieu basique, en présence de perruthénate de tétrapropylammonium, pour conduire aux composés de formule (V) : 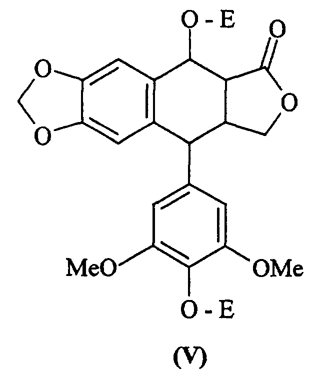 dans laquelle E est tel que défini précédemment,
composé de formule (V) qui est traité pendant plusieurs heures, par du fluorure de tétrabutylammonium, pour conduire aux composés de formule (I/a), cas particulier des composés de formule (I) : 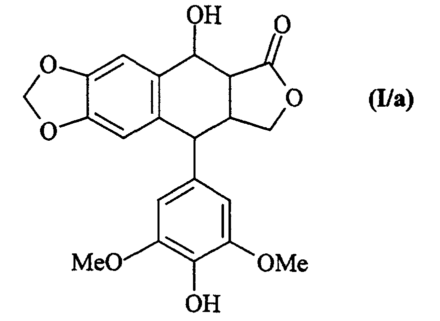 composé de formule (I/a) qui est soumis, en condition basique, à l'action d'un composé de formule (VII) :

R'₁ - X **(VII)**

dans laquelle R'₁ représente un groupement alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, alkylcarbonyle (C₁-C₆) linéaire ou ramifié, arylcarbonyle, arylalkylcarbonyle (C₁-C₆) linéaire ou ramifié, hétérocycloalkylcarbonyle, alkoxycarbonyle (C₁-C₆) linéaire ou ramifié, aryloxycarbonyle, arylalkoxycarbonyle (C₁-C₆) linéaire ou ramifié, alkylsulfonyle (C₁-C₆) linéaire ou ramifié, arylsulfonyle, ou arylalkylsulfonyle (C₁-C₆) linéaire ou ramifié, et X représente un atome d'hydrogène ou d'halogène, pour conduire aux composés de formule (I/b), cas particulier des composés de formule (I) : 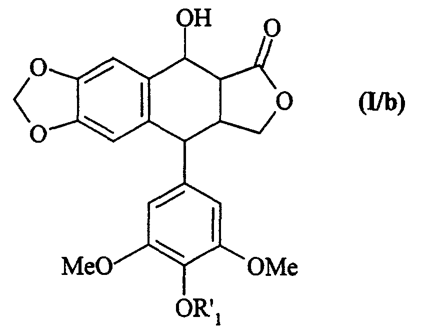 dans laquelle R'₁ est tel que défini précédemment,
l'ensemble des composés de formule (I/a) et (I/b) forment les composés de formule (I/c) : 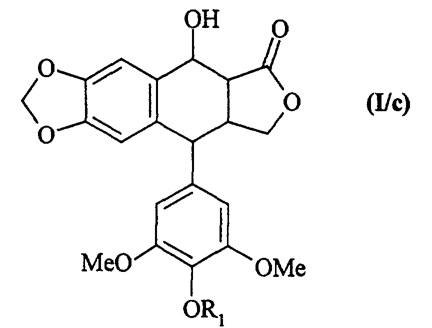 dans laquelle R₁ est tel que défini dans la formule (I),
composé de formule (I/c) qui est mis à réagir :
- **soit avec un composé de formule (VIII) :** 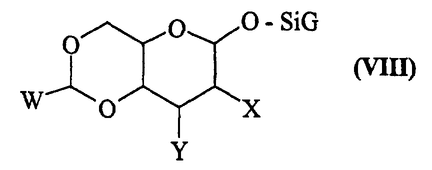 dans laquelle X, Y et W sont tels que définis dans la formule (I) et SiG représente un groupement protecteur silylé classiquement utilisé en chimie des sucres,
   en présence d'éthérate de trifluorure de bore, pour conduire aux composés de formule (I/d), cas particulier des composés de formule (I) : 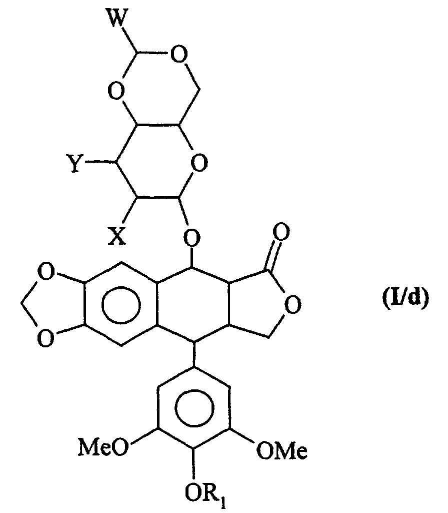 dans laquelle X, Y, W et R₁ sont tels que définis dans la formule (I),
- **soit, en présence d'une base, avec un composé de formule (IX) :** 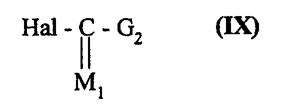 dans laquelle Hal représente un atome d'halogène, M₁ représente un atome d'oxygène ou de soufre, et G₂ représente un groupement T₂ ou -O-T₂ dans lesquels T₂ est tel que défini dans la formule (I),
   pour conduire aux composés de formule (I/e), cas particulier des composés de formule (I) : 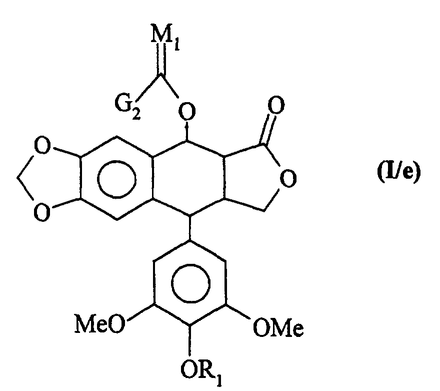 dans laquelle R₁ est tel que défini dans la formule (I) et, M₁ et G₂ sont tels que définis précédemment,
- **soit avec un composé de formule (X):**

   M₁ = C = N - T₂ **(X)**

   dans laquelle M₁ est tel que défini précédemment, et T₂ est tel que défini dans la formule (I),
   pour conduire aux composés de formule (I/f), cas particulier des composés de formule (I) : 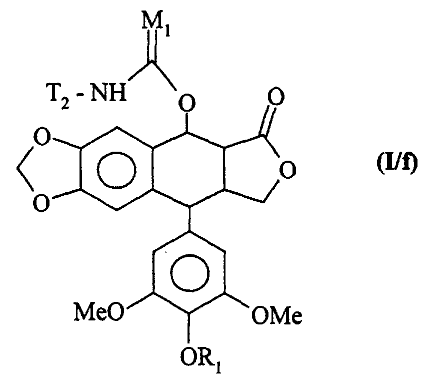 dans laquelle R_{1,} M₁ et T₂ sont tels que définis précédemment,
   composés de formule (I/f) qui peut être soumis à l'action d'un composé de formule (XI) :

   R'₂ - Hal **(XI)**

   dans laquelle Hal représente un atome d'halogène et R'₂ a les mêmes significations que R₂ dans la formule (I), excepté la valeur atome d'hydrogène,
   pour conduire aux composés de formule (I/g), cas particulier des composés de formule (I) : 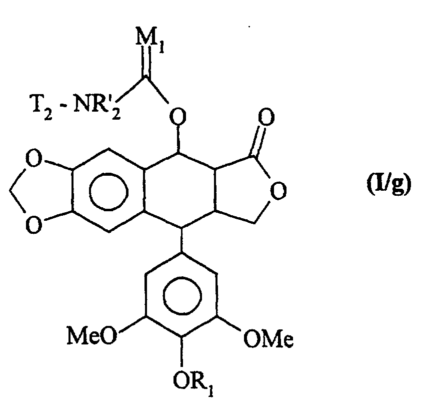 dans laquelle R₁, R'₂, T₂ et M₁ sont tels que définis précédemment,
- **soit avec l'azidure de sodium, pour conduire aux composés de formule (XIa) :** 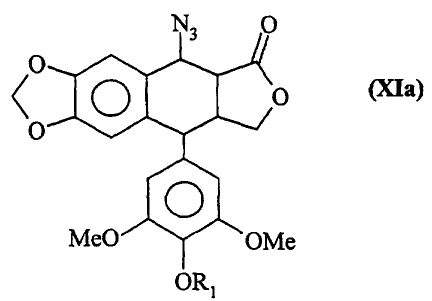 dans laquelle R₁ est tel que défini précédemment,
   composés de formule (XIa) dont on réduit la fonction azide en fonction amine primaire puis que l'on fait réagir :
   ◆ soit avec un composé de formule (X) telle que définie précédemment, pour conduire aux composés de formule (I/h), cas particulier des composés de formule (I) : 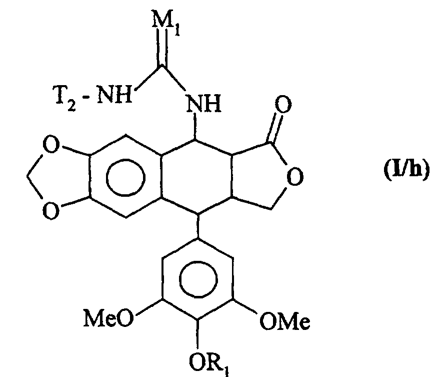 dans laquelle R₁, M₁ et T₂ sont tels que définis précédemment, composés de formule (I/h) dont les deux azotes de la fonction urée ou thiourée peuvent être aisément fonctionnalisés selon des conditions classiques de la synthèse organique, par traitement en milieu basique avec un composé de formule (XI) R'₂ - Hal telle que définie précédemment, pour conduire à une urée ou thiourée monofonctionnalisée ou bifonctionnalisée de formule -NR₂-C(M₁)-NR₂-T₂,
   ◆ soit avec un composé de formule (IX) telle que définie précédemment, pour conduire aux composés de formule (I/i), cas particulier des composés de formule (I) : 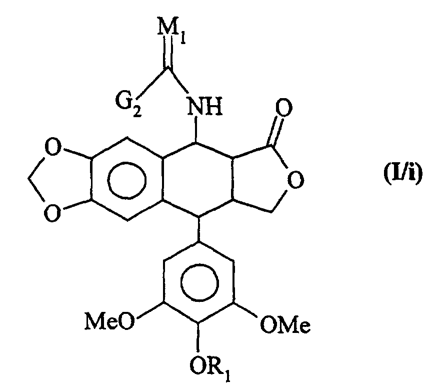 dans laquelle R₁, M₁ et G₂ sont tels que définis précédemment, composés de formule (I/i) dont l'atome d'azote peut être aisément fonctionnalisé selon des conditions classiques de la synthèse organique, par traitement en milieu basique avec un composé de formule (XI) telle que définie précédemment,
   ◆ soit avec un composé de formule (XII):

      Cl - SO₂ - T₃ **(XII)**

      dans laquelle T₃ a la même définition que dans la formule (I), pour conduire aux composés de formule (I/j), cas particulier des composés de formule (I) : 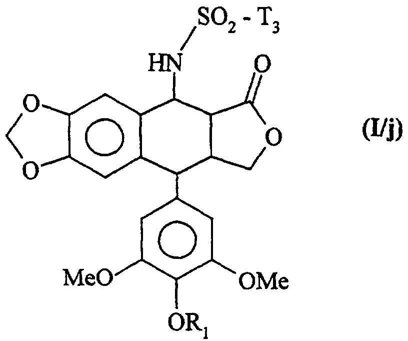 dans laquelle R₁ et T₃ sont tels que définis précédemment,
- **soit avec un composé de formule (XIII):**

   Hal - G₃ **(XIII)**

   dans laquelle Hal représente un atome d'halogène et G₃ représente un groupement choisi parmi -R'₂, -T₁-NR₃R₄, -T₁-NR₂-T'₁-NR₃R₄, dans lesquels R'₂, R₂, R₃, R₄, T₁ et T'₁ sont tels que définis précédemment,
   pour conduire aux composés de formule (I/k), cas particulier des composés de formule (I) : 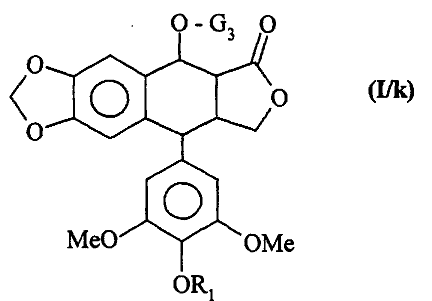 dans laquelle R₁ et G₃ sont tels que définis précédemment,
- **soit avec de l'acide chlorhydrique ou bromhydrique gazeux, pour conduire aux composés de formule (XIV):** 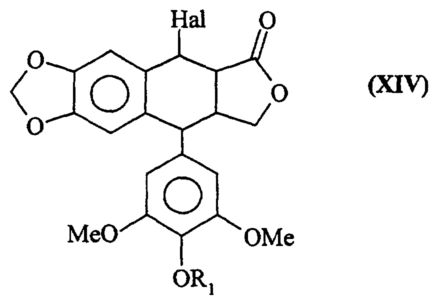 dans laquelle Hal représente un atome de chlore ou de brome et R₁ est tel que défini dans la formule (I),
   composés de formule (XIV) que l'on traite en milieu basique, avec un composé de formule (XV) ou de formule (XVbis) :

   H-N(R₂)-G₄ **(XV)** HN(R₄) - R₃ **(XVbis)**

   dans lesquelles R₂, R₃ et R₄ sont tels que définis dans la formule (I), et G₄ représente un groupement de formule -T₁-NR₃R₄, -T₁-OR₅,-T₁-CO₂R₆, ou T₁-CO-R₆, dans lesquels T₁, R₃, R₄, R₅ et R₆ ont les mêmes significations que dans la formule (I),
   pour conduire aux composés de formule (I/l) et (I/m), cas particulier des composés de formule (I), 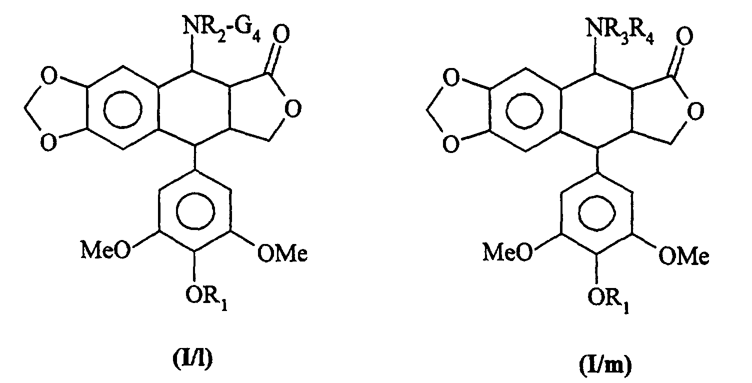 dans lesquels R₁, R₂, R₃, R₄ et G₄ sont tels que définis précédemment,
- **soit que l'on fait réagir, en présence d'éthérate de trifluorure de bore, avec un composé de formule (XVI) :**

   Me₃Si - A₁ - CH = CH₂ **(XVI)**

   dans laquelle A₁ représente une chaîne alkylène (C₁-C₅) linéaire ou ramifiée, pour conduire aux composés de formule (I/n), cas particulier des composés de formule (I) : 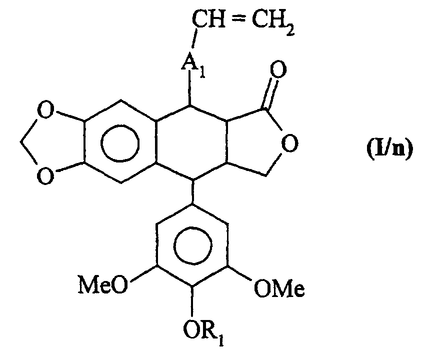 dans laquelle R₁ et A₁ sont tels que définis précédemment,
   composés de formule (I/n) que l'on traite par du tétraoxyde d'osmium en présence de N-méthylmorpholine oxyde, puis que l'on oxyde par action du tétraacétate de plomb, pour conduire aux composés de formule (XVII) : 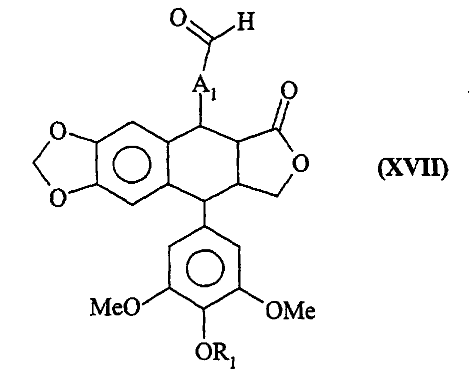 dans laquelle A₁ et R₁ sont tels que définis précédemment,
   composés de formule (XVII), qui est ensuite aisément traité par des méthodes classiques de réduction, d'oxydation, d'addition électrophile ou nucléophile, bien connues de l'homme de l'art versé dans le domaine de la synthèse organique, pour conduire aux composés de formule (I/o), cas particulier des composés de formule (I) : 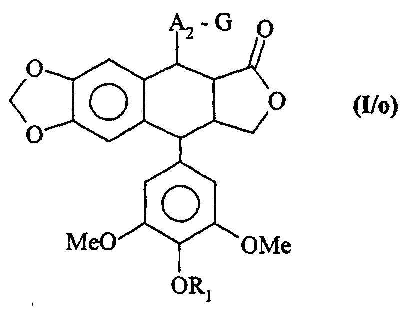 dans laquelle R₁ est tel que défini précédemment, G a la même définition que dans la formule (I), et A₂ a les mêmes significations que A dans la formule (I) excepté la définition liaison simple,
les composés (I/a) à (I/o) formant l'ensemble des composés de formule (I) de l'invention, que l'on purifie, le cas échéant, selon une technique classique de purification, qui peuvent, si on le désire, être séparés en leurs différents isomères selon une technique classique de séparation et que l'on transforme, si on le souhaite, en leurs sels d'addition à un acide ou à base pharmaceutiquement acceptable.

Les composés de formule (XIa), (XIV) et (XVII) sont utiles en tant qu'intermédiaires de synthèse pour l'obtention des composés de formule (I).

Les composés de formule (II), (VII), (VIII), (IX), (X), (XI), (XII), (XIII), (XV), (XVbis), et (XVI) sont soit des composés commerciaux, soit obtenus selon les méthodes classiques de synthèse organique.

Les composés de formule (I) présentent des propriétés antitumorales particulièrement intéressantes. Ils ont une excellente cytotoxicité in vitro sur des lignées cellulaires, issues de tumeurs murines et humaines, et sont actifs in vivo. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses isomères optiques, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent l'invention mais ne la limitent en aucune façon. Les produits de départ utilisés sont des produits connus ou préparés selon des modes opératoires connus.

Les structures des composés décrits dans les exemples ont été déterminées selon les techniques spectrophotométriques usuelles (infrarouge, résonance magnétique nucléaire, spectrométrie de masse, ...).

### EXEMPLE 1 : (5R,5aR,8aS,9R)-5-hydroxy-9-(4-hydroxy-3,5-diméthoxyphényl)-5,8,8a,9-tétrahydrofuro[3',4':6,7]naphto[2,3-d][1,3]dioxol-6-one

### STADE A: (5R,5aS,8aS,9R)-9-{[tert-butyl(diméthyl)silyl]oxy}-5-(4-(4-{[tert-butyl(diméthyl)silyl]oxy}-3,5-diméthoxyphényl)-5,8,8a,9-tétrahydrofuro [3',4':6,'7]naphto[2,3-d][1,3]dioxol-6-one

A une solution de 13,6 mmol de 4'-déméthylépipodophyllotoxine et 81,7 mmol de 2,6-lutidine dans 200 ml de dichlorométhane anhydre sont additionnées goutte à goutte, à 0°C et sous atmosphère inerte, 54,4 mmol de triflate de *tert*-butyldiméthylsilyle. Après 1 h 30 d'agitation à 0°C, 200 ml d'eau sont additionnés dans le milieu réactionnel. La phase organique est ensuite lavée jusqu'à neutralité, séchée, filtrée et concentrée sous pression réduite. Une chromatographie sur gel de silice (cyclohexane/acétate d'éthyle : 95/5) permet d'isoler le produit attendu.
***Point de fusion : 172-173°C***

### STADE B: [(5R,6S,7S,8R)-5{[tert-butyl(diméthyl)silyl]oxy}-8-(4-{[tert-butyl (diméthyl)silylloxy)-3,5-diméthoxyphényl)-7-(hydroxyméthyl)-5,6,7,8-tétrahydronaphto[2,3-d][1,3]dioxo-6-yl]méthanol

A une solution de 10,6 mmol du composé du stade A dans 200 ml de tétrahydrofurane anhydre, placée à 0°C sous argon, sont ajoutées 16 mmol d'hydrure de lithium et d'aluminium. Après 15 minutes d'agitation à 0°C puis à température ambiante, 0,6 ml d'eau, 0,6 ml d'une solution aqueuse de soude à 15 %, puis 1,8 ml d'eau sont ajoutés successivement au milieu réactionnel entraînant la formation d'un précipité. Après filtration de celui-ci, le filtrat est concentré sous pression réduite. Une chromatographie sur gel de silice (cyclohexane/acétate d'éthyle : 3/1) permet d'isoler le produit attendu.
***Point de fusion : 156-158°C***

### STADE C: (5R,5aR,8aS,9R)-5-([tert-butyl(diméthyl)silyl]oxy}-9-(4-{[tert-butyl (diméthyl)silyl]oxy}-3,5-diméthoxyphényl)-5,8,8a,9-tétrahydrofuro [3',4':6,7]naphto[2,3-d][1,3]dioxol-6(5aH)-one

A une solution de 3,95 mmol du composé du stade B dans 80 ml de dichlorométhane anhydre sont additionnés 1,97 g de tamis moléculaire 4Å, 11,8 mmol de N-oxyde de 4-méthylmorpholine, puis 0,4 mmol de perruthénate de tétrapropylammonium. Après 2 heures à température ambiante, le milieu réactionnel est filtré sur célite puis concentré sous pression réduite. Une chromatographie sur gel de silice (cyclohexane/acétate d'éthyle : 96/4 puis 95/5) permet d'isoler un mélange (1/1) constitué du produit attendu et du produit obtenu dans le stade A.
***Point de fusion: 193-194°C***

### STADE D: (5R,5aR,8aS,9R)-5-{[tert-butyl(diméthyl)silyl]oxy}-9-(4-hydroxy-3,5-diméthoxyphényl)-5,8,8a,9-tétrahydrofuro[3',4':6,7]naphto[2,3-d] [1,3]-dioxol-6(5aH)-one

A une solution de 2,78 mmol du mélange obtenu dans le stade C dans 60 ml de tétrahydrofurane anhydre, sont additionnées goutte à goutte sous argon, 4,3 mmol d'une solution 1M dans le tétrahydrofurane de fluorure de tétrabutylammonium. Après 20 minutes de réaction, 35 ml d'une solution aqueuse saturée en chlorure d'ammonium, 50 ml d'eau puis 100 ml d'acétate d'éthyle sont ajoutés au milieu réactionnel. Après extraction à l'acétate d'éthyle, les phases organiques sont lavées, séchées, filtrées puis concentrées sous pression réduite. Une chromatographie sur gel de silice (cyclohexane/acétate d'éthyle) permet d'isoler le produit attendu.
***Spectre de Masse (DIC*/*NH***_{***3***}***): m*/*z = 532 [M*+*NH***_{***4***}***]***^{**+**}

### STADE E : (5R,5aR,8aS,9R)-5-hydroxy-9-(4-hydroxy-3,5-diméthoxyphényl)-5,8,8a,9-tétrahydrofuro[3',4':6,7]naphto[2,3-d][1,3]dioxol-6-one

On procède comme dans le stade D précédent en utilisant comme substrat le produit obtenu dans le stade D et en maintenant la réaction sous agitation pendant 23 heures.
***Point de fusion : 129-131°C***
***Spectre de Masse (DIC*/*NH***_{***3***}***): m*/*z* = 418 *[M*+*NH***_{***4***}***]***^{**+**}

### EXEMPLE 2: 4-[(5R,5aS,8aR,9R)-9-hydroxy-8-oxo-5,5a,6,8,8a,9-hexahydrofuro [3',4':6,7]naphto[2,3-d][1,3]-dioxol-5-yl]-2,6-diméthozyphényl benzyl carbonate

A une solution de 0,25 mmol du composé de l'exemple 1 dans 10 ml de dichlorométhane anhydre, à 0°C et sous argon, sont additionnées successivement 0,55 mmol de triéthylamine et 0,37 mmol de chloroformiate de benzyle. Après 1 heure d'agitation, le milieu réactionnel est lavé à l'eau. La phase organique est ensuite séchée, filtrée puis concentrée sous pression réduite. Une chromatographie sur gel de silice (cyclohexane/acétate d'éthyle : 60/40) permet d'isoler le produit attendu.
***Point de fusion : 128-130°C***
***Spectre de masse Haute Résolution (DIC*/*NH***_{***3***}***)***:
***m*/*z (mesuré) = 552,1870 [M*+*NH***_{***4***}***]***^{**+**}
***m*/*z (calculé) = 552,1866 [M*+*NH***_{***4***}***]***^{**+**}

### EXEMPLE 3 : 4-((5R,5aS,8aR,9R)-9-{[(4-fluoroanilino)carbonyl]oxy}-8-oxo-5,5a,6,8,8a,9-hexahydrofuro[3',4':6,7]naphto[2,3-d][1,3]-dioxol-5-yl)-2,6-diméthoxyphényl benzyl carbonate

A une solution sous argon de 0,19 mmol du composé de l'exemple 2 dans 10 ml de dichlorométhane anhydre sont additionnées 0,038 mmol de 4-diméthylaminopyridine et 0,38 mmol de 2,6-lutidine, puis goutte à goutte, 0,27 mmol d'isocyanate de 4-fluorophényle. Après 22 heures de réaction, 0,15 mmol de 2,6-lutidine et 0,095 mmol d'isocyanate de 4-fluorophényle sont ajoutés au milieu réactionnel. Après 21 heures de réaction, le milieu réactionnel est hydrolysé et la phase organique est lavée, séchée, filtrée puis concentrée sous pression réduite. Une chromatographie sur gel de silice (cyclohexane/acétate d'éthyle : 3/1) permet d'isoler le produit attendu.
***Point de fusion : 202-204°C***
***Spectre de Masse (DIC*/*NH***_{***3***}***) : m*/*z = 689 [M*+*NH***_{***4***}***]***^{**+**}

### EXEMPLE 4 : (5R,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]dioxol-5-yl 4-fluorophénylcarbamate

A une solution de 0,1 mmol du composé de l'exemple 3 dans 8 ml d'acétate d'éthyle sont ajoutés 50 mg de Pd/C 5 %. Le milieu réactionnel est placé sous atmosphère d'hydrogène à pression atmosphérique. Après 1 heure d'agitation, le catalyseur est filtré sur célite et le filtrat est concentré sous pression réduite. Une chromatographie sur gel de silice (cyclohexane/acétate d'éthyle : 2/1) permet d'isoler le produit attendu.
***Point de fusion: 171-176°C***
***Spectre de Masse (DIC*/*NH***_{***3***}***) : m*/*z* = *555 [M*+*NH***_{***4***}***]***^{**+**}

### EXEMPLE 5: (5R,5aR,8aS,9R)-9-(4-{[(benzyloxy)carbonyl]oxy}-3,5-diméthoxyphényl-6-oxo-5,5a,6,8,8a,9-hexahydrofuro[3',4':6,7]naphto[2,3-d] [1,3]dioxol-5-yl 4-nitrophényl carbonate

A une solution de 3,23 mmol de chloroformiate de 4-nitrophényle dans 30 ml de dichlorométhane anhydre, sous argon, sont additionnées 3,61 mmol de pyridine, puis goutte à goutte, 0,95 mmol du composé de l'exemple 3 dilué dans 10 ml de dichlorométhane. Après 1 h 15 à température ambiante, le milieu réactionnel est hydrolysé. Après extraction au dichlorométhane, les phases organiques sont lavées, séchées, filtrées puis concentrées sous pression réduite. Une chromatographie sur gel de silice (cyclohexane/acétate d'éthyle : 3/1) permet d'isoler le produit attendu.
***Point de fusion*** ***: 125-130°C***
***Spectre de Masse (FAB***^{**+**}***): m*/*z = 698 [M-H]***^{**+**}

### EXEMPLE 6: 4-[(5R,5aS,8aR,9R)-9-({[[2-(diméthylamino)éthyl](méthyl)amino] carbonyl}oxy)-8-oxo-5,5a,6,8,8a,9-hexahydrofuro[3',4':6,7]naphto [2,3-d][1,3]dioxol-5-yl]-2,6-diméthoxyphényl benzyl carbonate

A une solution de 0,15 mmol du composé de l'exemple 5 dans 3 ml de dichlorométhane anhydre sont additionnées successivement 0,225 mmol de N,N,N'-triméthyléthylènediamine et 0,225 mmol de triéthylamine. Après 1 h 30 d'agitation, le milieu réactionnel est hydrolysé. La phase organique est ensuite lavée, séchée, filtrée puis concentrée sous pression réduite. Une chromatographie sur gel de silice (dichlorométhane/méthanol : 96/4) permet d'isoler le produit attendu.
***Point de fusion : 102-104°C***
***Spectre de Masse (DIC*/*NH***_{***3***}***): m*/*z = 663 [M*+*H]***^{**+**}

### EXEMPLE 7 : (5R,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]dioxol-5-yl 2-(diméthylamino)éthyl(méthyl)carbamate

Le composé de l'exemple 6 est soumis aux mêmes conditions opératoires que celles décrites dans l'exemple 4 permettant d'obtenir le produit attendu.
***Point de fusion : 133-135°C***
***Spectre de Masse Haute Résolution (FAB***^{**+**}***)*:**
***m*/*z (mesuré) = 529,2180 [M*+*H]***^{**+**}
***m*/*z (calculé) = 529,2186 [M*+*H]***^{**+**}

### EXEMPLE 8 : 4-{(5R,5aS,8aR,9R)-9-[({[2-(diméthylamino)éthyl]amino}carbonyl) oxy]-8-oxo-5,5a,6,8,8a,9-hexahydrofuro[3',4':6,7]naphto[2,3-d][1,3] dioxol-5-yl}-2,6-diméthoxyphényl benzyl carbonate

Le produit est obtenu selon les conditions opératoires décrites dans l'exemple 6 en utilisant comme substrat le produit de l'exemple 5 et comme réactif la N,N-diméthyléthylènediamine.
***Point de fusion : 113-116°C***
***Spectre de Masse Haute Résolution (FAB***^{**+**}***)*** :
***m*/*z (mesuré) = 649,2388 [M*+*H]***^{**+**}
***m*/*z (calculé) = 649,2397 [M*+*H]***^{**+**}

### EXEMPLE 9: (5R,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]dioxol-5-yl 2-(diméthylamino)éthylcarbamate

Le produit est obtenu selon les conditions opératoires décrites dans l'exemple 4 en utilisant comme substrat le composé de l'exemple 8.
***Point de fusion 140-142°C***
***Spectre de Masse Haute Résolution (FAB***^{**+**}***)*:**
***m*/*z (mesuré) = 515,2043 [M*+*H]***^{**+**}
***m*/*z (calculé) = 515,2030 [M*+*H]***^{**+**}

### EXEMPLE 10: 4-[(5R,5aS,8aR,9R)-9-({[[3-(diméthylamino)propyl] (méthyl)amino]carbonyl}-oxy)-8-oxo-5,5a,6,8,8a,9-hexahydrofuro [3',4':6,7]naphto[2,3-d][1,3]dioxol-5-yl]-2,6-diméthoxyphényl benzyl carbonate

Le produit est obtenu selon les conditions opératoires décrites dans l'exemple 6 en utilisant comme substrat le produit de l'exemple 5 et comme réactif la N,N,N'-triméthyl-1,3-propanediamine.
***Point de fusion : 124-126°C***
***Spectre de Masse Haute Résolution (FAB***^{**+**}***)*:**
***m*/*z (mesuré) = 677,2725 [M*+*NH***_{***4***}***]***^{**+**}
***m*/*z (calculé) = 677,2710 [M*+*NH***_{***4***}***]***^{**+**}

### EXEMPLE 11 : (5R,5aR,8aS,9R)-9-(4-hydroxy-3,5-diméthoxyphényl)-6-oxo-5,5a,6,8,8a,9-hexahydrofuro(3',4':6,7]naphto[2,3-d][1,3]dioxol-5-yl 3-(diméthylamino)propyl(méthyl)carbamate

Le produit est obtenu selon les conditions opératoires décrites dans l'exemple 4 en utilisant comme substrat le composé de l'exemple 10.
***Spectre de Masse Haute Résolution (FAB***^{**+**}***)*:**
***m*/*z (mesuré) = 542,5781 [M*+*NH***_{***4***}***]***^{**+**}
***m*/*z (calculé) = 542,5776 [M*+*NH***_{***4***}***]***^{**+**}

### EXEMPLE 12: 4-{(5R,5aS,8aR,9R)-9-[({[3-(diméthylamino)propyl] amino}carbonyl)oxy]-8-oxo-5,5a,6,8,8a,9-hexahydrofuro [3',4':6,7]naphto[2,3-d][1,3]dioxol-5-yl}-2,6-diméthoxyphényl benzyl carbonate

Le produit est obtenu selon les conditions opératoires décrites dans l'exemple 6 en utilisant comme substrat le produit de l'exemple 5 et comme réactif la N,N-diméthyl-1,3-propanediamine.
***Point de fusion: 102-104°C***
***Spectre de Masse Haute Résolution (FAB***^{**+**}***)***:
***m*/*z*** ***(mesuré) = 663,2539*** ***[M*+*H]***^{**+**}
***m*/*z*** ***(calculé) =*** ***663,2554 [M*+*H]***^{**+**}

### EXEMPLE 13 : (5R,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]dioxol-5-yl 3-(diméthylamino)propylcarbamate

Le produit est obtenu selon les conditions opératoires décrites dans l'exemple 4 en utilisant comme substrat le composé de l'exemple 12.
***Spectre de Masse Haute Résolution (FAB***^{**+**}***)***:
***m*/*z (mesuré) = 529,2180 [M*+*H]***^{**+**}
***m*/*z (calculé) = 529,2186 [M*+*H]***^{**+**}

### EXEMPLE 14: (5S,5aR,8aS,9R)-5-amino-9-(4-hydroxy-3,5-diméthoxyphényl)-5,8,8a,9-tétrahydrofuro[3',4':6,7]naphto[2,3-d][1,3]dioxol-6(5aH)-one

### STADE 1: (5S,5aR,8aS,9R)-5-azido-9-(4-hydroxy-3,5-diméthoxyphényl)-5,8,8a,9-tétrahydrofuro[3',4':6,7]naphto[2,3-d][1,3]dioxol-6(5aH)-one

A une solution de 8 mmol du composé de l'exemple 1 et 40 mmol d'azidure de sodium dans 16 ml de chloroforme sont ajoutés goutte à goutte 10,4 mmol d'acide trifluoroacétique. Après 15 minutes d'agitation, une solution aqueuse saturée de bicarbonate de sodium est alors ajoutée. La phase organique est lavée à l'eau, séchée, filtrée puis concentrée sous pression réduite. Une chromatographie sur gel de silice (dichlorométhane/acétate d'éthyle : 92/8) permet d'isoler le produit attendu.

### STADE 2: (5S,5aR,8aS,9R)-5-amino-9-(4-hydroxy-3,5-diméthoxyphényl)-5,8,8a,9-tétrahydrofuro[3',4':6,7]naphto[2,3-d][1,3]dioxol-6(5aH)-one

A une solution de 7 mmol du composé obtenu dans le stade 1 dans 160 ml d'acétate d'éthyle est ajouté 0,6 g de Pd/C à 10 %. Le mélange réactionnel est alors agité sous hydrogène pendant 16 heures, puis filtré sur célite. Après concentration sous pression réduite du filtrat, le résidu est chromatographié sur gel de silice (dichlorométhane/acétate d'éthyle : 90/10) permettant d'isoler le produit attendu.

### EXEMPLE 15 : 4-{(5R,5aS,8aR,9S)-9-[(4-fluorobenzoyl)amino]-8-oxo-5,5a,6,8,8a,9-hexahydrofuro[3',4':6,7]naphto[2,3-d][1,3]dioxol-5-yl] 2,6-diméthoxyphényl-4-fluorobenzoate

A une solution de 0,75 mmol du composé obtenu dans l'exemple 14 dans 12 ml de tétrahydrofurane anhydre sont additionnés 1,65 mmol de triéthylamine, puis 1,70 mmol du chlorure d'acide *para*-fluorobenzoïque. Après 2 heures d'agitation, le milieu réactionnel est évaporé. Le résidu est repris par du dichlorométhane et de l'eau. Après décantation et extraction au dichlorométhane, la phase organique est séchée, filtrée puis concentrée sous pression réduite. Une chromatographie sur gel de silice (dichlorométhane/acétone : 95/5) permet d'isoler le produit attendu.

### EXEMPLE 16 : N-[(5S,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]dioxol-5-yl] - 4-nitrobenzamide

### STADE 1: (5S,5aR,8aS,9R)-5-amino-9-[4-{[tert-butyl(diméthyl)silyl]oxy}-3,5-diméthoxyphényl]-5,8,8a,9-tétrahydrofuro[3',4':6,7]naphto[2,3-d] [1,3]dioxol-6(5aH)-one

Une solution de 7,4 mmol du composé de l'exemple 14, 58,8 mmol d'imidazole, et 13 mmol de chlorure de tert-butyldiméthylsilyle dans 225 ml de diméthylformamide anhydre est agité à température ambiante. Après 17 heures de réaction, le milieu est versé dans un mélange d'eau et d'éther. Après extraction à l'éther, les phases organiques rassemblées sont séchées, filtrées puis concentrées sous pression réduite. Le résidu est alors recristallisé dans un mélange heptane/benzène permettant d'isoler le produit attendu.

### STADE 2: N-[(5S,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]dioxol-5-yl] - 4-nitrobenzamide

A une solution de 0,6 mmol du composé obtenu dans le stade 1 dans 10 ml de dichlorométhane anhydre sont ajoutés 0,4 mmol de 1,4-diazabicyclo[2.2.2]octane puis 0,78 mmol d'acide *para*-nitrobenzoïque. Après 7 heures à température ambiante, le milieu réactionnel est évaporé et le résidu est chromatographié. Le produit isolé est ensuite solubilisé dans 50 ml de méthanol et de la résine Dowex 50X2-200 est ajoutée. Après 15 heures d'agitation à température ambiante, le milieu réactionnel est filtré puis concentré sous pression réduite, permettant d'isoler le produit attendu.

### EXEMPLE 17 : 4-{(5R,5a5,8aR,9S)-9-[(méthylsulfonyl)amino]-8-oxo-5,5a,6,8,8a,9-hexahydrofuro[3',4':6,7)naphto[2,3-d][1,3]-dioxol-5-yl}-2,6-diméthoxyphényl méthanesulfonate

On procède comme dans l'exemple 15 en utilisant comme réactif du chlorure d'acide méthanesulfonique.

### EXEMPLE 18 : N-{(5S,5aR,8aS,9R)-9-(4-hydroxy-3,5-diméthoxyphényl)-6-oxo-5,5a,6,8,8a,9-hexahydrofuro[3',4':6,7]naphto[2,3-d][1,3]-dioxol-5-yl}-4-(méthylsulfonyl)benzènesulfonamide

On procède comme dans l'exemple 16 en utilisant comme réactif du chlorure d'acide 4-méthanesulfonylbenzènesulfonique.

### EXEMPLE 19: N-[(5S,5aR,8aS,9R)-9-(4-hydroxy-3,5-diméthoaryphényl)-6-oxo-5,5a,6,8,8a,9-hexahydrofuro[3',4':6,7]naphto[2,3-d][1,3]-dioxol-5-yl]-2-thiophènesulfonamide

On procède comme dans l'exemple 16 en utilisant comme réactif du chlorure d'acide 2-thiophènesulfonique.

### EXEMPLE 20: (5S,5aR,8aS,9R)-9-(4-hydroxy-3,5-diméthoxyphényl)-5-propyl-5,8,8a,9-tétrahydrofuro(3',4':6,7]naphto[2,3-d][1,3]-dioxol-6(5aH)-one

### STADE 1: 4-[(5R,5aS,8aS,9S)-8-oxo-9-propényl-5,5a,6,8,8a,9-hexahydrofuro[3',4':6,7]naphto[2,3-d] [1,3]dioxol-5-yl]-2,6-diméthoxyphényl benzyl carbonate

A une solution de 7,95 mmol du composé de l'exemple 2 dans 80 ml de dichlorométhane anhydre sont ajoutés 32,6 mmol de triméthylallylsilane puis 21,5 mmol d'éthérate de trifluorure de bore. Après 2 heures d'agitation à 0°C puis 30 minutes à température ambiante, 32,7 mmol de pyridine sont additionnées au milieu réactionnel. Après traitement à l'acide chlorhydrique dilué, puis lavage à l'eau, la phase organique est décantée puis la phase aqueuse est extraite au dichlorométhane. Les phases organiques rassemblées sont séchées, filtrées puis concentrées sous pression réduite. Une chromatographie sur gel de silice (dichlorométhane/acétone: 96/4) permet d'isoler le produit attendu.

### STADE 2: (5S,5aR,8aS,9R)-9-(4-hydroxy-3,5-diméthoxyphényl)-5-propyl-5,8,8a,9-tétrahydrofuro[3',4':6,7]naphto[2,3-d][1,3]-dioxol-6(5aH)-one

On procède comme dans l'exemple 4 en utilisant comme substrat le produit obtenu dans le stade 1 précédent.

### EXEMPLE 21 : (5R,5aS,8aS,9R)-5-(2,3-dihydroxypropyl)-9-(4-hydroxy-3,5-diméthoxyphényl)-5,8,8a,9-tétrahydrofuro[3',4':6,7]naphto[2,3-d] [1,3]dioxol-6(5aH)-one

### STADE 1: 4-[(5R,5aS,8aS,9R)-9-(2,3-dihydroxypropyl)-8-oxo-5,5a,6,8,8a,9-hexahydrofuro[3',4':6,7]naphto[2,3-d][1,3]dioxol-5-yl]-2,6-diméthoxyphényl benzyl carbonate

A une solution de 1,92 mmol du composé obtenu dans le stade 1 de l'exemple 20 et de 1,92 mmol de N-méthylmorpholine oxyde dans 15 ml d'acétone anhydre est ajouté 0,17 mmol de tétraoxyde d'osmium. Après 2 heures d'agitation à température ambiante, un mélange de glace et d'une solution aqueuse saturée de bisulfite de sodium est versé dans le milieu réactionnel. Après une heure d'agitation, le milieu est extrait au dichlorométhane. La phase organique est lavée avec de l'acide chlorhydrique 1N, puis avec de l'eau, puis séchée, filtrée et concentrée sous pression réduite permettant d'isoler le produit attendu.

### STADE 2: (5R,5aS,8aS,9R)-5-(2,3-dihydroxypropyl)-9-(4-hydroxy-3,5-diméthoxyphényl)-5,8,8a,9-tétrahydrofuro[3',4':6,7]naphto[2,3-d] [1,3]dioxol-6(5aH)-one

On procède comme dans l'exemple 4 en utilisant comme substrat le produit obtenu dans le stade 1 précédent.

### EXEMPLE 22 : (5R,5aR,8aS,9R)-5-{[(2S,6R,8R)-8-amino-2-méthylhexahydropyrano[3,2-d][1,3]-dioxin-6-yl]oxy}-9-(4-hydroxy-3,5-diméthoxyphényl)-5,8,8a,9-tétrahydrofuro[3',4':6,7]naphto[2,3-d][1,3]dioxol-6 (5aH)-one

### STADE 1: 4-((5R,5aS,8aR,9R)-9-{[(2S,6R,8R)-8-azido-2-méthylhexahydropyrano[3,2-d][1,3]dioxin-6-yl] oxy}-8-oxo-5,5a,6,8,8a,9-hexahydrofuro[3',4':6,7]naphto[2,3-d][1,3]dioxol-5-yl)-2,6-diméthoxyphényl benzyl carbonate

A une solution refroidie à -35°C de 1,64 mmol du composé de l'exemple 2 et de 1,64 mmol de (*tert*-butyl-dimétllylsilyl)-3-azido-2,3-didéoxy-4,6-*O*-éthylidène-β-D-*ribo*-hexapyranoside dans 20 ml de dichlorométhane anhydre sont ajoutés 2 g de tamis moléculaire 4Å en poudre et 2,8 mmol de triméthylsilyltrifluorométhanesulfonate. Après 12 heures, le milieu réactionnel est dilué par du dichlorométhane, puis lavé par un tampon citrate de pH 5 et de l'eau. Après extraction, les phases organiques sont séchées, filtrées puis concentrées sous pression réduite. Une chromatographie sur gel de silice (cyclohexane/acétate d'éthyle : 2/1) permet d'isoler le produit attendu.

### STADE 2: (5R,5aR,8aS,9R)-5-{[(2S,6R,8R)-8-amino-2-méthylhexahydropyrano[3,2-d][1,3]-dioxin-6-yl]oxy}-9-(4-hydroxy-3,5-diméthoxyphényl)-5,8,8a,9-tétrahydrofuro[3',4':6,7]naphto[2,3-d][1,3]dioxol-6 (5aH)-one

On procède comme dans l'exemple 4 en utilisant comme substrat le produit obtenu dans le stade 1 précédent.

### EXEMPLE 23 : 4-((5R,5aS,8aR,9R)-9-{[(4-fluoroanilino)carbonyl]oxy}-8-oxo-5,5a,6,8,8a,9-hexahydrofuro[3',4':6,7] naphto[2,3-d][1,3]dioxol-5-yl)-2,6-diméthoxyphényl 4-nitrophényl carbonate

A une solution de 0,83 mmol de chloroformate de *para*-nitrophényle dans 20 ml de dichlorométhane anhydre, sont additionnés, sous argon, 1,07 mmol de pyridine, puis goutte à goutte une solution de 0,59 mmol du composé de l'exemple 4 dans 5 ml de dichlorométhane. Après 1 heure d'agitation, le milieu réactionnel est hydrolysé par addition d'une solution aqueuse saturée en NaHCO₃, puis les phases organiques extraites sont lavées, neutralisées, séchées, filtrées et concentrées sous pression réduite. Une chromatographie sur gel de silice (cyclohexane/acétate d'éthyle : 3/1) permet d'isoler le produit attendu.

### EXEMPLE 24 : 4-((5R,5aS,8aR,9R)-9-{[(4-fluoroanilino)carbonyl]oxy}-8-oxo-5,5a,6,8,8a,9-hexahydrofuro[3',4':6,7]naphto[2,3-d][1,3]-dioxol-5-yl)-2,6-diméthoxyphényl 4-pipéridinyl-1-pipéridinecarboxylate

A une solution de 0,56 mmol du composé de l'exemple 23 dans 20 ml de dichlorométhane anhydre sont additionnées, sous atmosphère d'argon, 0,87 mmol de triéthylamine et 0,87 mmol de 4-pipéridino-pipéridine. Après 2 heures, 20 ml d'eau sont ajoutés au milieu réactionnel. Après extraction au dichlorométhane, lavage, séchage, filtration, et concentration sous pression réduite des phases organiques, une chromatographie sur gel de silice (dichlorométhane, méthanol : 95/5) permet d'isoler le produit attendu.

### ETUDE PHARMACOLOGIOUE DES COMPOSES DE L'INVENTION

### EXEMPLE 25 : Activité in vitro

La leucémie murine L1210 et le carcinome du colon humain HT-29 ont été utilisés in vitro. Les cellules sont cultivées dans du milieu de culture RPMI 1640 complet contenant 10 % de sérum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 heures (L1210) ou 96 heures (HT-29). Le nombre de cellules viables est ensuite quantifié par un essai colorimétrique, le Microculture Tetrazolium Assay (J. Carmichael et al., Cancer Res.; 47, 936-942, (1987)). Les résultats sont exprimés en IC₅₀, concentration en cytotoxique qui inhibe à 50 % la prolifération des cellules traitées.
Lors de ce test, le composé de l'exemple 9 présente un IC₅₀ de 88 nM.

### EXEMPLE 26 : Action sur le cycle cellulaire

Les cellules L1210 sont incubées pendant 21 heures à 37°C en présence de différentes concentrations en produit testés. Les cellules sont ensuite fixées par de l'éthanol à 70 % (v/v), lavées deux fois dans du PBS et incubées 30 minutes à 20°C dans du PBS contenant 100 µg/ml de RNAse et 50 µg/ml d'iodure de propidium. Les résultats sont exprimés en pourcentage des cellules accumulées en phase G2+M après 21 heures par rapport au témoin (témoin : 20 %). Le composé de l'exemple 9 induit une accumulation à 75 % des cellules en phase G2+M après 21 heures à une concentration de 1 µM.

## Revendications

1. Composés de formule (I) : 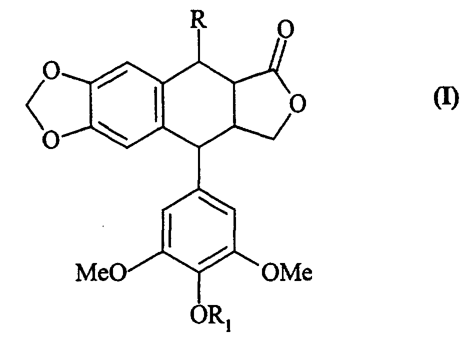 dans laquelle :
R représente :
- *un groupement de formule (i) :* 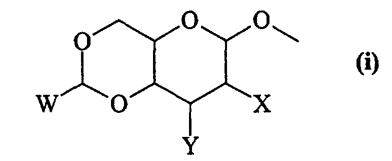 dans laquelle :
X et Y, identiques ou différents, représentent un groupement choisi parmi hydrogène, hydroxy, alkoxy (C₁-C₆) linéaire ou ramifié, amino, alkylamino (C₁-C₆) linéaire ou ramifié, ou dialkylamino (C₁-C₆) linéaire ou ramifié,
W représente un atome d'hydrogène, un groupement alkyle (C₁-C₆) linéaire ou ramifié, aryle ou hétéroaryle,
- *ou un groupement de formule (ii) :*
-A-G **(ii)**
dans laquelle :
A représente une liaison simple ou une chaîne alkylène (C₁-C₆) linéaire ou ramifiée, éventuellement substituée par un ou plusieurs groupements, identiques ou différents, choisis parmi halogène et hydroxy, et comportant éventuellement une insaturation,
G représente un groupement choisi parmi atome d'hydrogène, cycloalkyle, hétérocycloalkyle, aryle, hétéroaryle, -OR₂, -O-T₁-NR₃R₄, -O-T₁-NR₂-T'₁-NR₃R₄, -NR₃R₄, - NR₂-T₁-NR₃R₄, -NR₂-T₁-OR₅, -NR₂-T₁-CO₂R₆, -NR₂-T₁-C(O)R₆, -C(O)-NR₃R₄, -C(O)-NR₂-T₂, -O-C(O)-T₂, -O-C(S)-T₂, -NR₂-C(O)-T₂, -NR₂-C(S)-T₂, -O-C(O)-O-T₂, -O-C(O)-NR₂-T₂, -O-C(S)-O-T₂, -O-C(S)-NR₂-T₂, -NR₂-C(O)-O-T₂, -NR₂-C(O)-NR₆-T₂, -NR₂-C(S)-O-T₂, -NR₂-C(S)-NR₆-T₂, et -NR₂-SO₂-T₃ dans lesquels :
R₂ représente un atome d'hydrogène, un groupement alkyle (C₁-C₆) linéaire ou ramifié, aryle ou arylalkyle (C₁-C₆) linéaire ou ramifié,
T₁, T'₁, identiques ou différents, représentent une chaîne alkylène (C₁-C₆) linéaire ou ramifié,
R₃, R₄, identiques ou différents, indépendamment l'un de l'autre, représentent :
- un atome d'hydrogène, un groupement alkyle (C₁-C₆) linéaire ou ramifié, (éventuellement substitué par un ou plusieurs groupements hydroxy), aryle, arylalkyle (C₁-C₆) linéaire ou ramifié, cycloalkyle, hétéroarylalkyle (C₁-C₆) linéaire ou ramifié, hétéroaryle, hétérocycloalkyle, hétérocycloalkylalkyle (C₁-C₆) linéaire ou ramifié,
- ou forment ensemble avec l'atome d'azote qui les porte un hétérocycle de 5 à 7 chaînons, saturé ou insaturé, monocyclique, et comportant éventuellement un second hétéroatome choisi parmi oxygène et azote, ledit hétérocycle étant éventuellement substitué, par un ou plusieurs groupements choisis parmi halogène, hydroxy, alkyle (C₁-C₆) linéaire ou ramifié, et hétérocycle,
R₅ représente un groupement alkyle (C₁- C₆) linéaire ou ramifié, aryle, ou arylalkyle (C₁-C₆) linéaire ou ramifié,
R₆ représente un atome d'hydrogène, un groupement alkyle (C₁-C₆) linéaire ou ramifié, aryle, ou arylalkyle (C₁-C₆) linéaire ou ramifié,
T₂ représente un groupement choisi parmi atome d'hydrogène, alkyle (C₁-C₆) linéaire ou ramifié (éventuellement substitué par un ou plusieurs atomes d'halogène), aryle, arylalkyle (C₁-C₆) linéaire ou ramifié, cycloalkyle, cycloalkylalkyle (C₁-C₆) linéaire ou ramifié, hétérocycloalkyle et hétérocycloalkylalkyle (C₁-C₆) linéaire ou ramifié, ou T₂ représente une chaîne alkylène (C₁-C₆) linéaire ou ramifié, ladite chaîne étant substituée par un ou plusieurs groupements, identiques ou différents, choisis parmi -NR₃R₄, -OR₂, -CO₂R₆, -NR₂-C(O)R₆, -NR₂-CO₂R₆, -C(O)R₆, -C(O)NR₃R₄, -NR₂-T₁-NR₃R₄, -NR₂-T₁-OR₆, et -O-T₁-NR₃R₄ dans lesquels R₂, R₃, R₄, R₆ et T₁ sont tels que définis précédemment,
T₃ représente un groupement choisi parmi alkyle (C₁-C₂₀) linéaire ou ramifié (éventuellement substitué par un ou plusieurs groupements choisis parmi halogène, -OR₂, -NR₂R₆, nitro, cyano, azide), aryle, arylalkyle (C₁-C₆) linéaire ou ramifié, cycloalkyle, cycloalkylalkyle (C₁-C₆) linéaire ou ramifié, hétérocycloalkyle, hétérocycloalkylalkyle (C₁-C₆) linéaire ou ramifié, hétéroaryle et hétéroarylalkyle (C₁-C₆) linéaire ou ramifié,
R₁représente un groupement choisi parmi hydrogène, alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, alkylcarbonyle (C₁-C₆) linéaire ou ramifié, arylcarbonyle, arylalkylcarbonyle (C₁-C₆) linéaire ou ramifié, hétérocycloalkylcarbonyle, alkylsulfonyle (C₁-C₆) linéaire ou ramifié, arylsulfonyle, arylalkylsulfonyle (C₁-C₆) linéaire ou ramifié, phosphonique, alkoxycarbonyle (C₁-C₆) linéaire ou ramifié, aryloxycarbonyle, et arylalkoxy(C₁-C₆)carbonyle linéaire ou ramifié,
leurs isomères ainsi que leurs sels d'addition à un acide ou à une base pharmaceutiquement acceptable,
étant entendu que :
* *par aryle,* on comprend un groupement phényle, biphényle, naphtyle, dihydronaphtyle, tétrahydronaphtyle, indényle ou indanyle, chacun de ces groupements comportant éventuellement une ou plusieurs substitutions, identiques ou différentes, choisies parmi halogène, hydroxy, alkyle (C₁-C₆) linéaire ou ramifié, alkoxy (C₁-C₆) linéaire ou ramifié, cyano, nitro, amino, alkylamino (C₁-C₆) linéaire ou ramifié, dialkylamino (C₁-C₆) linéaire ou ramifié, alkylsulfonyle (C₁-C₆) linéaire ou ramifié, alkylsulfonylamino (C₁-C₆) linéaire ou ramifié, carboxy, alkoxycarbonyle (C₁-C₆) linéaire ou ramifié, aryloxycarbonyle, arylalkoxycarbonyle (C₁-C₆) linéaire ou ramifié, hydroxyalkyle (C₁-C₆) linéaire ou ramifié, trihalogénoalkyle (C₁-C₆) linéaire ou ramifié, méthylènedioxy, éthylènedioxy, morpholinyle, pipéridyle, pipérazinyle, alkylcarbonyloxy (C₁-C₆) linéaire ou ramifié, et alkylcarbonyle (C₁-C₆) linéaire ou ramifié,
* *par hétéroaryle,* on comprend un monocycle aromatique, un bicycle aromatique, ou un bicycle dont l'un des cycles est aromatique et l'autre cycle est partiellement hydrogéné, de 5 à 12 chaînons, comportant au sein du système cyclique un, deux ou trois hétéroatomes, identiques ou différents, choisis parmi oxygène, azote et soufre, ledit hétéroaryle pouvant éventuellement être substitué par les mêmes substitutions que celles décrites dans le cas du groupement aryle,
* *par cycloalkyle,* on comprend un groupement monocyclique ou bicyclique, saturé ou insaturé mais sans caractère aromatique, comportant de 3 à 10 atomes de carbone, étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, hydroxyalkyle (C₁-C₆) linéaire ou ramifié, amino, alkylamino (C₁-C₆) linéaire ou ramifié, dialkylamino (C₁-C₆) linéaire ou ramifié, pipéridyle, pipérazinyle, et morpholinyle,
* *par hétérocycloalkyle,* on comprend un cycloalkyle tel que défini précédemment, comportant un ou deux hétéroatomes, identiques ou différents, choisis parmi oxygène, azote et soufre, ledit hétérocycloalkyle étant éventuellement substitué par un ou plusieurs substituants tels que ceux décrits dans le cas du groupement cycloalkyle,
étant entendu également que R₁ ne représente pas un groupement méthyle lorsque R représente un groupement -A-G dans lequel A représente une liaison simple et G représente un atome d'hydrogène.

2. Composés de formule (I) selon la revendication 1 **caractérisés en ce que** R₁ représente un groupement choisi parmi atome d'hydrogène, groupement alkyle (C₁-C₆) linéaire ou ramifié, alkoxycarbonyle (C₁-C₆) linéaire ou ramifié, aryloxycarbonyle et arylalkoxycarbonyle (C₁-C₆) linéaire ou ramifié, leurs isomères ainsi que leurs sels d'addition à un acide ou à une base pharmaceutiquement acceptable.

3. Composés de formule (I) selon l'une quelconque des revendications 1 et 2 **caractérisés en ce que** R₁ représente un atome d'hydrogène, leurs isomères ainsi que leurs sels d'addition à un acide ou à une base pharmaceutiquement acceptable.

4. Composés de formule (I) selon la revendication 1 **caractérisés en ce que** R représente un groupement de formule (i), telle que définie dans la formule (I), dans laquelle :
◆ X représente un groupement amino, alkylamino (C₁-C₆) linéaire ou ramifié ou dialkylamino (C₁-C₆) linéaire ou ramifié et Y représente un groupement hydroxy, ou X et Y, identiques, représentent chacun un groupement hydroxy, ou X représente un atome d'hydrogène et Y représente alors un groupement amino, alkylamino (C₁-C₆) linéaire ou ramifié, ou dialkylamino (C₁-C₆) linéaire ou ramifié, et
◆ W représente un groupement méthyle ou 2-thiényle, leurs isomères ainsi que leurs sels d'addition à un acide ou à une base pharmaceutiquement acceptable.

5. Composés de formule (I) selon l'une quelconque des revendications 1 et 4 **caractérisés en ce que** R représente un groupement de formule (i) telle que : 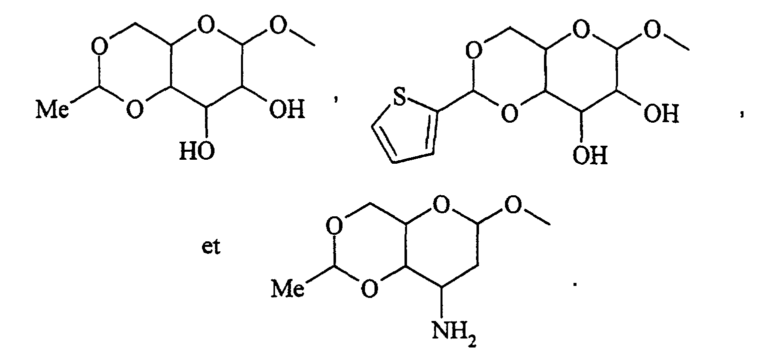 leurs isomères ainsi que leurs sels d'addition à un acide ou à une base pharmaceutiquement acceptable.

6. Composés de formule (I) selon la revendication 1 **caractérisés en ce que** R représente un groupement de formule (ii), telle que définie dans la formule (I), dans laquelle :
◆ A représente une liaison simple, et
◆ G représente un groupement choisi parmi O-T₁-NR₃R₄, -O-T₁-NR₂-T'₁-NR₃R₄, -NR₂-T₁-NR₃R₄, NR₃R₄, -NR₂-T₁-OR₅, -O-C(O)-NR₂-T₂ et -NR₂-SO₂-T₃ dans lesquels T₁, T'₁, T₂, T₃, R₂, R₃, R₄ et R₅ sont tels que définis dans la formule (I),
leurs isomères ainsi que leurs sels d'addition à un acide ou à une base pharmaceutiquement acceptable.

7. Composés de formule (I) selon l'une quelconque des revendications 1 et 6 **caractérisés en ce que** R représente un groupement de formule (ii), telle que définie dans la formule (I), dans laquelle :
◆ A représente une liaison simple, et
◆ G représente un groupement -O-C(O)-NR₂-T₂ dans lequel R₂ et T₂ sont tels que définis dans la formule (I), et de façon avantageuse, R₂ représente un atome d'hydrogène ou un groupement alkyle (C₁-C₆) linéaire ou ramifié et T₂ représente un groupement aryle éventuellement substitué ou une chaîne alkylène (C₁-C₆) linéaire ou ramifiée substituée par un groupement -NR₃R₄ dans lequel R₃ et R₄ sont tels que définis dans la formule (I),
leurs isomères ainsi que leurs sels d'addition à un acide ou à une base pharmaceutiquement acceptable.

8. Composés de formule (I) selon l'une quelconque des revendications 1 et 6 **caractérisés en ce que** R représente un groupement de formule (ii), telle que définie dans la formule (I), dans laquelle :
◆ A représente une liaison simple, et
◆ G représente un groupement -NR₂-SO₂-T₃ dans lequel R₂ et T₃ sont tels que définis dans la formule (I), et de façon avantageuse, R₂ représente un atome d'hydrogène ou un groupement alkyle (C₁-C₆) linéaire ou ramifié et T₃ représente un groupement alkyle (C₁-C₆) linéaire ou ramifié, aryle ou hétéroaryle,
leurs isomères ainsi que leurs sels d'addition à un acide ou à une base pharmaceutiquement acceptable.

9. Composés de formule (I) selon la revendication 1 qui sont :
- le 9-(4-hydroxy-3,5-diméthoxyphényl)-6-oxo-5,5a,6,8,8a,9-hexahydrofuro [3',4':6,7] naphto[2,3-*d*][1,3]dioxol-5-yl 4-fluorophénylcarbamate,
- le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 2-(diméthylamino)éthyl(méthyl)carbamate,
- le 9-(4-hydroxy-3,5-diméthoxyphényl)-6-oxo-5,5a,6,8,8a,9-hexahydrofuro[3',4':6,7] naphto[2,3-*d*][1,3]-dioxol-5-yl 2-(diméthylamino)éthylcarbamate,
- le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 3-(diméthylamino)propyl(méthyl)carbamate,
- et le 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 3-(diméthylamino)propylcarbamate,
leurs isomères ainsi que leurs sels d'addition à un acide ou à une base pharmaceutiquement acceptable.

10. Composés de formule (I) selon la revendication 1 qui sont :
- le (5*R*,5a*R*,8a*S*,9*R*) 9-(4-hydroxy-3,5-diméthoxyphényl)-6-oxo-5,5a,6,8,8a,9-hexahydrofuro[3',4':6,7]naphto[2,3-*d*][1,3]dioxol-5-yl 4-fluorophénylcarbamate,
- le (5*R*,5a*S*,8a*R*,9*R*) 9-(4-hydroxy-3,5-diméthoxyphényl)-6-oxo-5,5a,6,8,8a,9-hexahydrofuro[3',4':6,7]naphto[2,3-*d*][1,3]dioxol-5-yl 2-(diméthylamino)éthyl(méthyl) carbamate,
- le (5*R*,5a*R*,8a*S*,9*R*) 9-(4-hydroxy-3,5-diméthoxyphényl)-6-oxo-5,5a,6,8,8a,9-hexahydrofuro[3',4':6,7]naphto[2,3-*d*][1,3]-dioxol-5-yl 2-(diméthylamino) éthylcarbamate,
- le (5*R*,5a*R*,8a*S*,9*R*) 9-(4-hydroxy-3,5-diméthoxyphényl)-6-oxo-5,5a,6,8,8a,9-hexahydrofuro[3',4':6,7]naphto[2,3-*d*][1,3]dioxol-5-yl 3-(diméthylamino)propyl (méthyl)carbamate,
- et le (5*R*,5a*S*,8a*R*,9*R*) 9-(4-hydroxy-3,5-diméthoxyphényl)-6-oxo-5,5a,6,8,8a,9-hexahydrofuro[3',4':6,7] naphto[2,3-*d*][1,3]dioxol-5-yl 3-(diméthylamino) propylcarbamate.

11. Procédé de préparation des composés de formule (I), **caractérisé en ce que** soit utilisé comme produit de départ un composé de formule (II) : 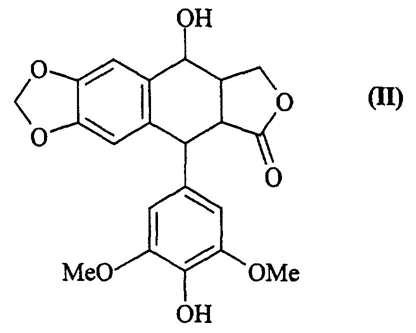 composé de formule (II) dont les fonctions hydroxy sont protégées par un groupement protecteur silylé classiquement utilisé en chimie organique, selon des conditions opératoires usuelles de la synthèse organique, pour conduire au composé de formule (III) : 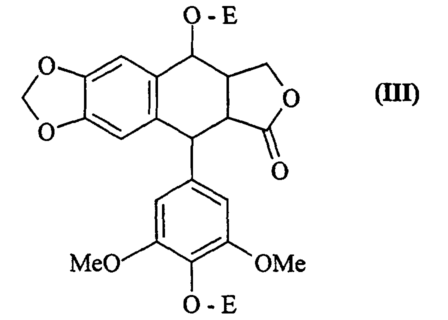 dans laquelle E représente un groupement protecteur silylé,
composé de formule (III) qui est traité par un agent de réduction pour conduire au composé de formule (IV) : 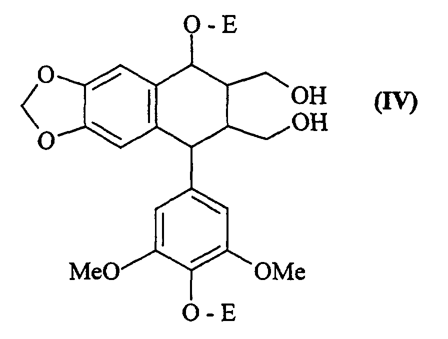 dans laquelle E est tel que défini précédemment,
composé de formule (IV) qui est mis à réagir, en milieu basique, en présence de perruthénate de tétrapropylammonium, pour conduire aux composés de formule (V) : 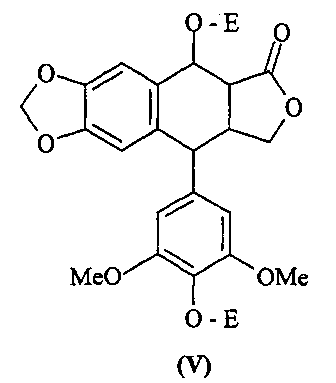 dans laquelle E est tel que défini précédemment,
composé de formule (V) qui est traité pendant plusieurs heures, par du fluorure de tétrabutylammonium, pour conduire aux composés de formule (I/a), cas particulier des composés de formule (I) : 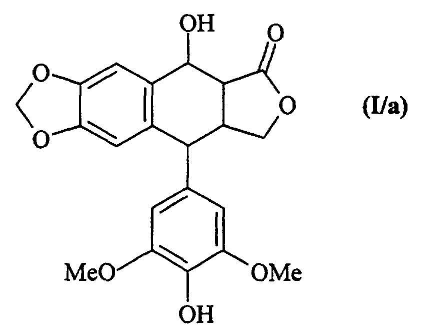 composé de formule (I/a) qui est soumis, en condition basique, à l'action d'un composé de formule (VII):
R'₁ - X **(VII)**
dans laquelle R'₁ représente un groupement alkyle (C₁-C₆) linéaire ou ramifié, aryle, arylalkyle (C₁-C₆) linéaire ou ramifié, hétéroaryle, hétéroarylalkyle (C₁-C₆) linéaire ou ramifié, alkylcarbonyle (C₁-C₆) linéaire ou ramifié, arylcarbonyle, arylalkylcarbonyle (C₁-C₆) linéaire ou ramifié, hétérocycloalkylcarbonyle, alkoxycarbonyle (C₁-C₆) linéaire ou ramifié, aryloxycarbonyle, arylalkoxycarbonyle (C₁-C₆) linéaire ou ramifié, alkylsulfonyle (C₁-C₆) linéaire ou ramifié, arylsulfonyle, ou arylalkylsulfonyle (C₁-C₆) linéaire ou ramifié, et X représente un atome d'hydrogène ou d'halogène, pour conduire aux composés de formule (I/b), cas particulier des composés de formule (I) : 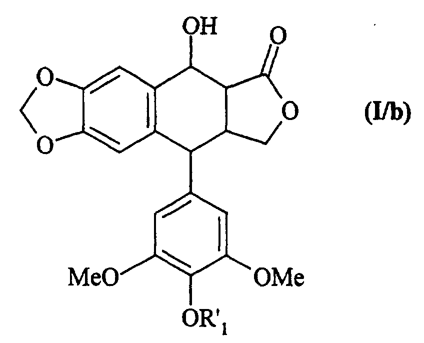 dans laquelle R'₁ est tel que défini précédemment,
l'ensemble des composés de formule (I/a) et (I/b) forment les composés de formule (I/c) : 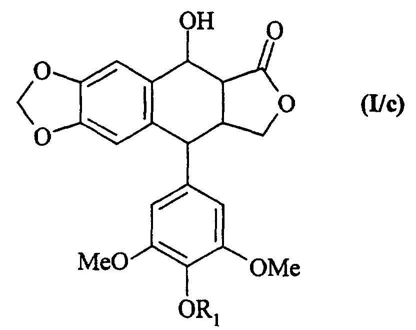 dans laquelle R₁ est tel que défini dans la formule (I),
composé de formule (I/c) qui est mis à réagir :
• **soit avec un composé de formule (VIII) :** 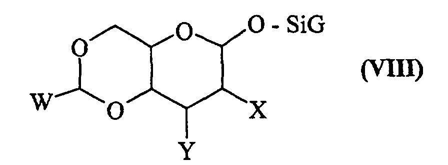 dans laquelle X, Y et W sont tels que définis dans la formule (I) et SiG représente un groupement protecteur silylé classiquement utilisé en chimie des sucres,
en présence d'éthérate de trifluorure de bore, pour conduire aux composés de formule (I/d), cas particulier des composés de formule (I) : 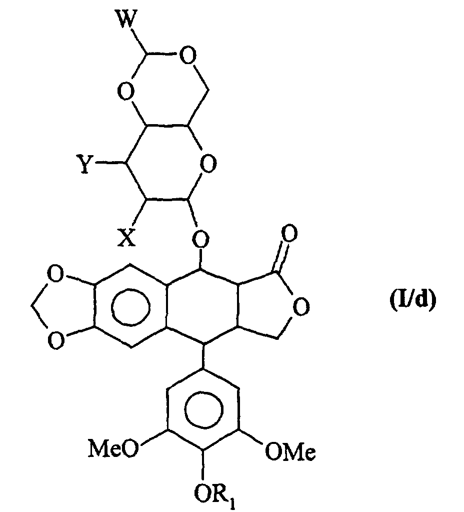 dans laquelle X, Y, W et R₁ sont tels que définis dans la formule (I),
• **soit, en présence d'une base, avec un composé de formule (IX) :** 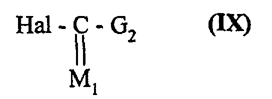 dans laquelle Hal représente un atome d'halogène, M₁ représente un atome d'oxygène ou de soufre, et G₂ représente un groupement T₂ ou -O-T₂ dans lesquels T₂ est tel que défini dans la formule (I),
pour conduire aux composés de formule (I/e), cas particulier des composés de formule (I) : 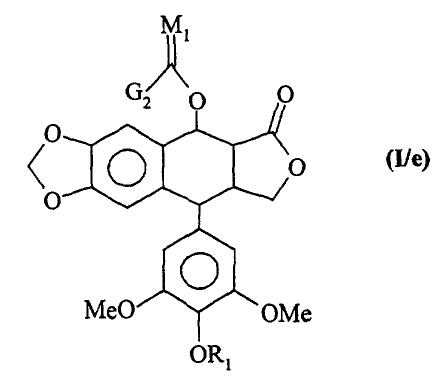 dans laquelle R₁ est tel que défini dans la formule (I) et, M₁ et G₂ sont tels que définis précédemment,
• **soit avec un composé de formule (X) :**
M₁ = C = N - T₂ **(X)**
dans laquelle M₁ est tel que défini précédemment, et T₂ est tel que défini dans la formule (I),
pour conduire aux composés de formule (I/f), cas particulier des composés de formule (I) : 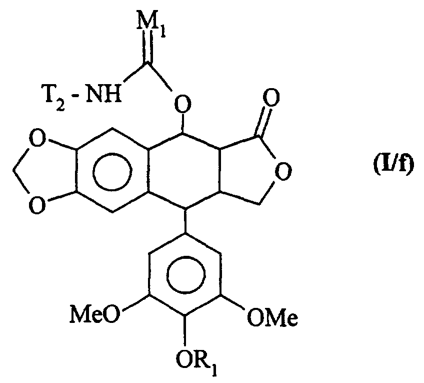 dans laquelle R₁, M₁ et T₂ sont tels que définis précédemment,
composés de formule (I/f) qui peut être soumis à l'action d'un composé de formule (XI) :
R'₂ - Hal **(XI)**
dans laquelle Hal représente un atome d'halogène et R'₂ a les mêmes significations que R₂ dans la formule (I), excepté la valeur atome d'hydrogène,
pour conduire aux composés de formule (I/g), cas particulier des composés de formule (I) : 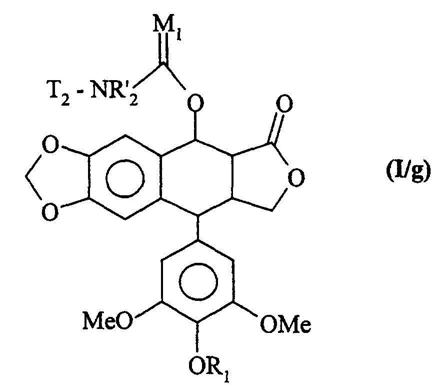 dans laquelle R₁, R'₂, T₂ et M₁ sont tels que définis précédemment,
• **soit avec l'azidure de sodium, pour conduire aux composés de formule (XIa) :** 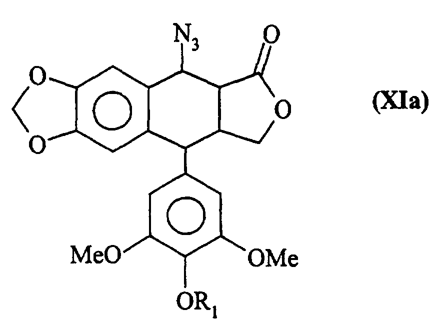 dans laquelle R₁ est tel que défini précédemment,
composés de formule (XIa) dont on réduit la fonction azide en fonction amine primaire puis que l'on fait réagir :
◆ soit avec un composé de formule (X) telle que définie précédemment, pour conduire aux composés de formule (I/h), cas particulier des composés de formule (I): 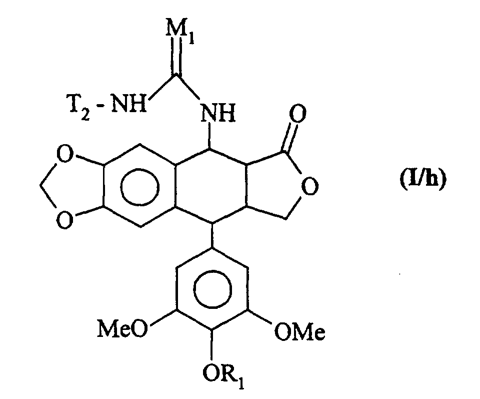 dans laquelle R₁, M₁ et T₂ sont tels que définis précédemment, composés de formule (I/h) dont les deux azotes de la fonction urée ou thiourée peuvent être aisément fonctionnalisés selon des conditions classiques de la synthèse organique, par traitement en milieu basique avec un composé de formule (XI) R'₂ - Hal telle que définie précédemment, pour conduire à une urée ou thiourée monofonctionnalisée ou bifonctionnalisée de formule -NR₂-C(M₁)-NR₂-T₂,
◆ soit avec un composé de formule (IX) telle que définie précédemment, pour conduire aux composés de formule (I/i), cas particulier des composés de formule (I): 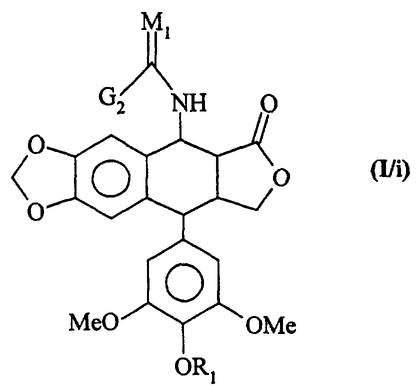 dans laquelle R₁, M₁ et G₂ sont tels que définis précédemment, composés de formule (I/i) dont l'atome d'azote peut être aisément fonctionnalisé selon des conditions classiques de la synthèse organique, par traitement en milieu basique avec un composé de formule (XI) telle que définie précédemment,
◆ soit avec un composé de formule (XII):
C₁ - SO₂ - T₃ **(XII)**
dans laquelle T₃ a la même définition que dans la formule (I), pour conduire aux composés de formule (I/j), cas particulier des composés de formule (I) : 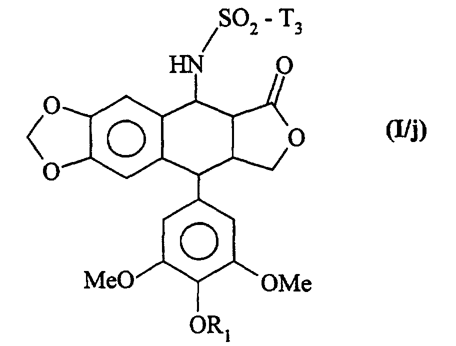 dans laquelle R₁ et T₃ sont tels que définis précédemment,
• **soit avec un composé de formule (XIII) :**
Hal - G₃ **(XIII)**
dans laquelle Hal représente un atome d'halogène et G₃ représente un groupement choisi parmi -R'₂, -T₁-NR₃R₄, -T₁-NR₂-T'₁-NR₃R_{4,} dans lesquels R'₂, R₂, R₃, R₄, T₁ et T'₁ sont tels que définis précédemment, pour conduire aux composés de formule (I/k), cas particulier des composés de formule (I) : 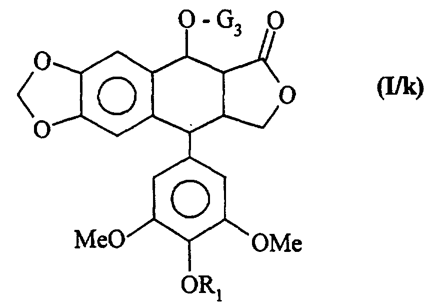 dans laquelle R₁ et G₃ sont tels que définis précédemment,
• **soit avec de l'acide chlorhydrique ou bromhydrique gazeux, pour conduire aux composés de formule (XIV) :** 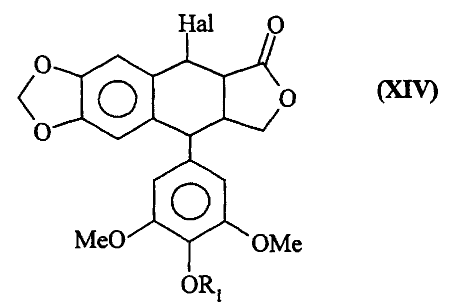 dans laquelle Hal représente un atome de chlore ou de brome et R₁ est tel que défini dans la formule (I),
composés de formule (XIV) que l'on traite en milieu basique, avec un composé de formule (XV) ou de formule (XVbis) :
H - N(R₂) - G₄ **(XV)** HN(R₄) - R₃ **(XVbis)**
dans lesquelles R₂, R₃ et R₄ sont tels que définis dans la formule (I), et G₄ représente un groupement de formule -T₁-NR₃R₄, -T₁-OR₅, -T₁-CO₂R₆, ou T₁-CO-R₆, dans lesquels T₁, R₃, R₄, R₅ et R₆ ont les mêmes significations que dans la formule (I),
pour conduire aux composés de formule (I/l) et (I/m), cas particulier des composés de formule (I), 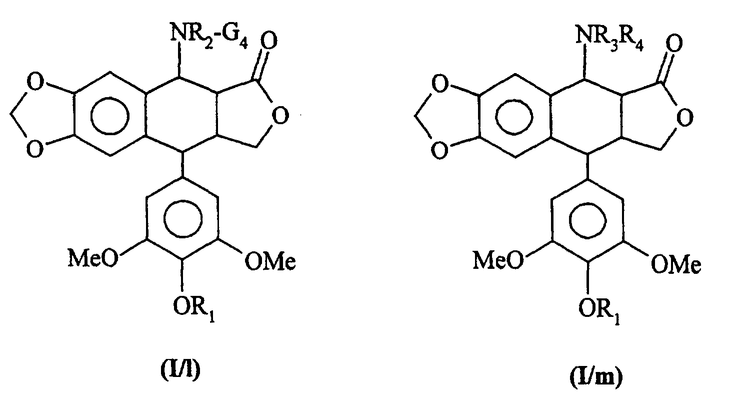 dans lesquels R₁, R₂, R₃, R₄ et G₄ sont tels que définis précédemment,
• **soit que l'on fait réagir, en présence d'éthérate de trifluorure de bore, avec un composé de formule (XVI) :**
Me₃Si - A₁ - CH = CH₂ **(XVI)**
dans laquelle A₁ représente une chaîne alkylène (C₁-C₅) linéaire ou ramifiée, pour conduire aux composés de formule (I/n), cas particulier des composés de formule (I) : 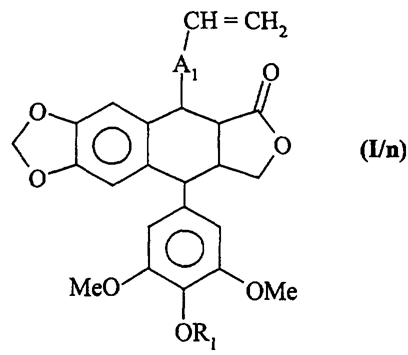 dans laquelle R₁ et A₁ sont tels que définis précédemment,
composés de formule (I/n) que l'on traite par du tétraoxyde d'osmium en présence de N-méthylmorpholine oxyde, puis que l'on oxyde par action du tétraacétate de plomb, pour conduire aux composés de formule (XVII) : 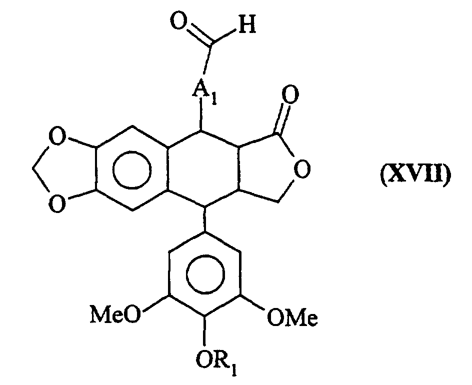 dans laquelle A₁ et R₁ sont tels que définis précédemment,
composés de formule (XVII), qui est ensuite aisément traité par des méthodes classiques de réduction, d'oxydation, d'addition électrophile ou nucléophile, bien connues de l'homme de l'art versé dans le domaine de la synthèse organique, pour conduire aux composés de formule (I/o), cas particulier des composés de formule (I) : 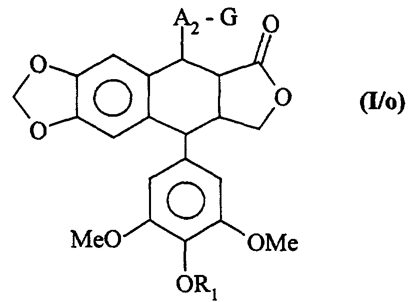 dans laquelle R₁ est tel que défini précédemment, G a la même définition que dans la formule (I), et A₂ a les mêmes significations que A dans la formule (I) excepté la définition liaison simple,
les composés (I/a) à (I/o) formant l'ensemble des composés de formule (I) de l'invention, que l'on purifie, le cas échéant, selon une technique classique de purification, qui peuvent, si on le désire, être séparés en leurs différents isomères selon une technique classique de séparation et que l'on transforme, si on le souhaite, en leurs sels d'addition à un acide ou à base pharmaceutiquement acceptable.

12. Composés de formule (XIa), (XIV) et (XVII) telles que décrites dans la revendication 11, utiles en tant qu'intermédiaires de synthèse pour l'obtention des composés de formule (I).

13. Compositions pharmaceutiques contenant comme principe actif au moins un composé selon l'une quelconque des revendications 1 à 10, seul ou en combinaison avec un ou plusieurs excipients ou véhicules inertes, non toxiques, pharmaceutiquement acceptables.

14. Compositions pharmaceutiques selon la revendication 13, utile en tant que médicament, contenant au moins un principe actif selon l'une quelconque des revendications 1 à 10, utiles dans le traitement du cancer.

## Patentansprüche

1. Verbindungen der Formel (I): 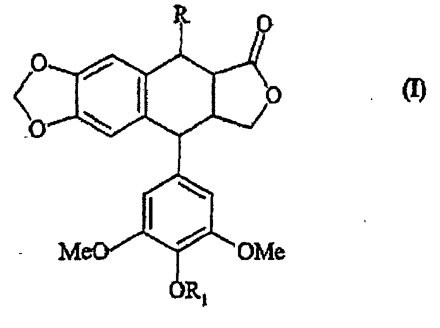 in der:
R:
- *eine Gruppe der Formel (i):* 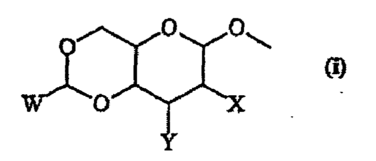 in der:
X und Y, die gleichartig oder verschieden sind, eine Gruppe ausgewählt aus Wasserstoff, Hydroxy, geradkettigem oder verzweigtem (C₁-C₆)-Alkoxy, Amino, geradkettigem oder verzweigtem (C₁-C₆)-Alkylamino oder geradkettigem oder verzweigtem Di-(C₁-C₆)-alkylamino und
W ein Wasserstoffatom, eine geradkettige oder verzweigte (C₁-C₆)-Alkylgruppe, Arylgruppe oder Heteroarylgruppe bedeuten,
- *oder eine Gruppe der Formel (ii):*
- A - G **(ii)**
in der:
A eine Einfachbindung oder eine geradkettige oder verzweigte (C₁-C₆)-Alkylenkette bedeutet, die gegebenenfalls durch eine oder mehrere gleichartige oder verschiedenartige Gruppen ausgewählt aus Halogen und Hydroxy substituiert ist und gegebenenfalls eine Unsättigung aufweist,
G eine Gruppe bedeutet ausgewählt aus Wasserstoff, Cycloalkyl, Heterocycloalkyl, Aryl, Heteroaryl, -OR₂, -O-T₁-NR₃R₄, -O-T₁-NR₂-T'₁-NR₃R₄, -NR₃R₄, -NR₂-T₁-NR₃R₄, -NR₂-T₁-OR₅, -NR₂-T₁-CO₂R₆, -NR₂-T₁-C(O)R₆, -C(O)-NR₃R₄, -C(O)-NR₂-T₂, -O-C(O)-T₂, -O-C(S)-T₂, -NR₂-C(O)-T₂, -NR₂-C(S)-T₂, -O-C(O)-O-T₂, -O-C(O)-NR₂-T₂, -O-C(S)-O-T₂, -O-C(S)-NR₂-T₂, -NR₂-C(O)-O-T₂, -NR₂-C(O)-NR₆-T₂, -NR₂-C(S)-O-T₂, -NR₂-C(S)-NR₆-T₂ und -NR₂-SO₂-T₃, in denen:
R₂ ein Wasserstoffatom, eine geradkettige oder verzweigte (C₁-C₆)-Alkylgruppe, eine Arylgruppe oder eine geradkettige oder verzweigte Aryl-(C₁-C₆)-alkylgruppe darstellt,
T₁, T'₁, die gleichartig oder verschieden sind, eine geradkettige oder verzweigte (C₁-C₆)-Alkylenkette darstellen,
R₃, R₄, die gleichartig oder verschieden sind, unabhängig voneinander:
- ein Wasserstoffatom, eine geradkettige oder verzweigte (C₁-C₆)-Alkylgruppe (die gegebenenfalls durch eine oder mehrere Hydroxygruppen substituiert ist), Arylgruppe, geradkettige oder verzweigte Aryl-(C₁-C₆)-alkylgruppe, Cycloalkylgruppe, geradkettige oder verzweigte Heteroaryl-(C₁-C₆)-alkylgruppe, Heteroarylgruppe, Heterocycloalkylgruppe oder geradkettige oder verzweigte Heterocycloalkyl-(C₁-C₆)-alkylgruppe bedeuten,
- oder gemeinsam mit dem sie tragenden Stickstoffatom einen gesättigten oder ungesättigten monocyclischen Heterocyclus mit 5 bis 7 Kettengliedern bilden, der gegebenenfalls ein zweites Heteroatom ausgewählt aus Sauerstoff und Stickstoff aufweist, wobei der Heterocyclus gegebenenfalls durch eine oder mehrere Gruppen ausgewählt aus Halogen, Hydroxy, geradkettigem oder verzweigtem (C₁-C₆)-Alkyl und Heterocyclus substituiert ist,
R₅ eine geradkettige oder verzweigte (C₁-C₆)-Alkylgruppe, Arylgruppe oder geradkettige oder verzweigte Aryl-(C₁-C₆)-alkylgruppe darstellt,
R₆ ein Wasserstoffatom, eine geradkettige oder verzweigte (C₁-C₆)-Alkylgruppe, Arylgruppe oder geradkettige oder verzweigte Aryl-(C₁-C₆)-alkylgruppe darstellt,
T₂ eine Gruppe darstellt ausgewählt aus Wasserstoff, geradkettigem oder verzweigtem (C₁-C₆)-Alkyl (welches gegebenenfalls durch ein oder mehrere Halogenatome substituiert ist), Aryl, geradkettigem oder verzweigtem Aryl-(C₁-C₆)-alkyl, Cycloalkyl, geradkettigem oder verzweigtem Cycloalkyl-(C₁-C₆)-alkyl, Heterocycloalkyl und geradkettigem oder verzweigtem Heterocycloalkyl-(C₁-C₆)-alkyl, oder T₂ eine geradkettige oder verzweigte (C₁-C₆)-Alkylenkette darstellt, welche Kette durch eine oder mehrere gleichartige oder verschiedenartige Gruppen substituiert ist, ausgewählt aus -NR₃R₄, -OR₂, -CO₂R₆, -NR₂-C(O)R₆, -NR₂-CO₂R₆, -C(O)R₆, -C(O)NR₃R₄, -NR₂-T₁-NR₃R₄, -NR₂-T₁-OR₆ und -O-T₁-NR₃R₄, in denen R₂, R₃, R₄, R₆ und T₁ die oben angegebenen Bedeutungen besitzen,
T₃ eine Gruppe ausgewählt aus geradkettigem oder verzweigtem (C₁-C₂₀)-Alkyl (das gegebenenfalls durch eine oder mehrere Gruppen ausgewählt aus Halogen, -OR₂, -NR₂R₆, Nitro, Cyano, Azid substituiert ist), Aryl, geradkettigem oder verzweigtem Aryl-(C₁-C₆)-alkyl, Cycloalkyl, geradkettigem oder verzweigtem Cycloalkyl-(C₁-C₆)-alkyl, Heterocycloalkyl, geradkettigem oder verzweigtem Heterocycloalkyl-(C₁-C₆)-alkyl, Heteroaryl und geradkettigem oder verzweigtem Heteroaryl-(C₁-C₆)-alkyl darstellt, und
R₁ eine Gruppe bedeutet ausgewählt aus Wasserstoff, geradkettigem oder verzweigtem (C₁-C₆)-Alkyl, Aryl, geradkettigem oder verzweigtem Aryl-(C₁-C₆)-alkyl, Heteroaryl, geradkettigem oder verzweigtem Heteroaryl-(C₁-C₆)-alkyl, geradkettigem oder verzweigtem (C₁-C₆)-Alkylcarbonyl, Arylcarbonyl, geradkettigem oder verzweigtem Aryl-(C₁-C₆)-alkylcarhonyl, Heterocycloalkylcarbonyl, geradkettigem oder verzweigtem (C₁-C₆)-Alkylsulfonyl, Arylsulfonyl, geradkettigem oder verzweigtem Aryl-(C₁-C₆)-alkylsulfonyl, Phosphonyl, geradkettigem oder verzweigtem (C₁-C₆)-Alkoxycarbonyl, Aryloxycarbonyl und geradkettigem oder verzweigtem Aryl-(C₁-C₆)-alkoxycarbonyl bedeutet,
deren Isomere sowie deren Additionssalze mit einer pharmazeutisch annehmbaren Säure oder Base,
wobei es sich versteht, daß man:
* *unter Aryl* eine Phenyl-, Biphenyl-, Naphthyl-, Dihydronaphthyl-, Tetrahydronaphthyl-, Indenyl- oder Indanylgruppe versteht, wobei jede dieser Gruppen gegebenenfalls einen oder mehrere gleichartige oder verschiedenartige Substituenten aufweist, ausgewählt aus Halogen, Hydroxy, geradkettigem oder verzweigtem (C₁-C₆,)-Alkyl, geradkettigem oder verzweigtem (C₁-C₆)-Alkoxy, Cyano, Nitro, Amino, geradkettigem oder verzweigtem (C₁-C₆)-Alkylamino, geradkettigem oder verzweigtem Di-(C₁-C₆)-alkylamino, geradkettigem oder verzweigtem (C₁-C₆)-Alkylsulfonyl, geradkettigem oder verzweigtem (C₁-C₆)-Alkylsulfonylamino, Carboxy, geradkettigem oder verzweigtem (C₁-C₆)-Alkoxycarbonyl, Aryloxycarbonyl, geradkettigem oder verzweigtem Arylalkoxy-(C₁-C₆)-carbonyl, geradkettigem oder verzweigtem (C₁-C₆)-Hydroaryalkyl, geradkettigem oder verzweigtem (C₁-C₆)-Trihalogenalkyl, Methylendioxy, Ethylendioxy, Morpholinyl, Piperidyl, Piperazinyl, geradkettigem oder verzweigtem (C₁-C₆)-Alkylcarbonyloxy und geradkettigem oder verzweigtem (C₁-C₆)-Alkylcarbonyl,
* *unter Heteroaryl* einen aromatischen Monocyclus, aromatischen Bicyclus oder einen Bicyclus, bei dem einer der Cyclen aromatisch ist und der andere teilweise hydriert, mit 5 bis 12 Kettengliedern versteht, der im Kern des cyclischen Systems eines, zwei oder drei gleichartige oder verschiedenartige Heteroatome aufweist, ausgewählt aus Sauerstoff, Stickstoff und Schwefel, wobei der Heteroarylrest gegebenenfalls durch die gleichen Substituenten substituiert sein kann, wie sie für die Arylgruppe beschrieben worden sind,
* *unter Cycloalkyl* eine monocyclische oder bicyclische, gesättigte oder ungesättigte Gruppe ohne aromatischen Charakter mit 3 bis 10 Kohlenstoffatomen versteht, die gegebenenfalls durch eine oder mehrere gleichartige oder verschiedenartige Gruppen substituiert ist, ausgewählt aus Halogen, geradkettigem oder verzweigtem (C₁-C₆)-Alkyl, geradkettigem oder verzweigtem (C₁-C₆)-Trihalogenalkyl, Hydroxy, geradkettigem oder verzweigtem (C₁-C₆)-Hydroxyalkyl, Amino, geradkettigem oder verzweigtem (C₁-C₆)-Alkylamino, geradkettigem oder verzweigtem Di-(C₁-C₆)-alkylamino. Piperidyl, Piperazinyl und Morpholinyl,
* *unter Heterocycloalkyl* eine Cycloalkylgruppe versteht, wie sie oben definiert worden ist, die ein oder zwei gleichartige oder verschiedenartige Heteroatome aufweist, ausgewählt aus Sauerstoff, Stickstoff und Schwefel, wobei der Heterocycloalkylrest gegebenenfalls durch einen oder mehrere Substituenten substituiert sein kann, wie sie bezüglich der Cycloalkylgruppe beschrieben worden sind,
mit der weiteren Maßgabe, daß R₁ keine Methylgruppe darstellt, wenn R eine Gruppe -A-G bedeutet, in der A eine Einfachbindung und G ein Wasserstoffatom darstellen.

2. Verbindungen der Formel (I) nach Anspruch 1, **dadurch gekennzeichnet, daß** R₁ eine Gruppe ausgewählt aus Wasserstoff, geradkettigem oder verzweigtem (C₁-C₆)-Alkyl, geradkettigem oder verzweigtem (C₁-C₆)-Alkoxycarbonyl, Aryloxycarbonyl und geradkettigem oder verzweigtem Aryl-(C₁-C₆)-alkoxycarbonyl bedeutet, deren Isomere sowie deren Additionssalze mit einer pharmazeutisch annehmbaren Säure oder Base.

3. Verbindungen der Formel (I) nach einem der Ansprüche 1 und 2, **dadurch gekennzeichnet, daß** R₁ ein Wasserstoffatom bedeutet, deren Isomere sowie deren Additionssalze mit einer pharmazeutisch annehmbaren Säure oder Base.

4. Verbindungen der Formel (I) nach Anspruch 1, **dadurch gekennzeichnet, daß** R eine Gruppe der Formel (i) bedeutet, wie sie bezüglich der Formel (I) definiert ist, in der:
◆ X eine Aminogruppe, geradkettige oder verzweigte (C₁-C₆)-Alkylaminogruppe oder eine geradkettige oder verzweigte Di-(C₁-C₆)-alkylaminogruppe darstellt und Y eine Hydroxygruppe bedeutet, oder X und Y, die gleichartig sind, jeweils eine Hydroxygruppe bedeuten, oder X ein Wasserstoffatom und Y eine Aminogruppe, eine geradkettige oder verzweigte (C₁-C₆)-Alkylaminogruppe oder eine geradkettige oder verzweigte Di-(C₁-C₆)-alkylaminogruppe bedeutet, und
◆ W eine Methyl- oder 2-Thienylgruppe darstellt, deren Isomere sowie deren Additionssalze mit einer pharmazeutisch annehmbaren Säure oder Base.

5. Verbindungen der Formel (I) nach einem der Ansprüche 1 und 4, **dadurch gekennzeichnet, daß** R eine der folgenden Gruppen der Formel (i) bedeutet: 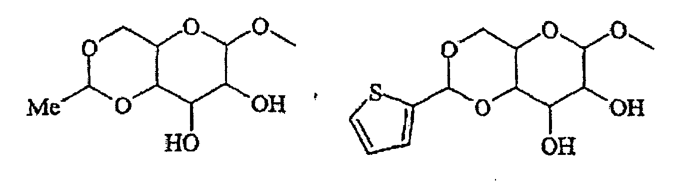 und 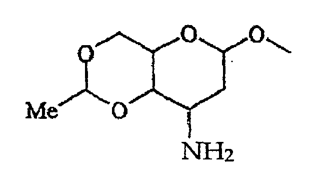 deren Isomere sowie deren Additionssalze mit einer pharmazeutisch annehmbaren Säure oder Base.

6. Verbindungen der Formel (I) nach Anspruch 1, **dadurch gekennzeichnet, daß** R eine Gruppe der Formel (ii) darstellt, wie sie bezüglich der Formel (I) definiert worden ist, in der:
◆ A eine Einfachbindung darstellt und
◆ G eine Gruppe bedeutet ausgewählt aus O-T₁-NR₃R₄, -O-T₁-NR₂-T'₁-NR₃R₄, -NR₂-T₁-NR₃R₄, -NR₃-R₄, -NR₂-T₁-OR₅, -O-C(O)-NR₂-T₂ und -NR₂-SO₂-T₃, in denen T₁, T'₁, T₂, T₃, R₂, R₃, R₄ und R₅ die bezüglich der Formel (I) angegebenen Bedeutungen besitzen,
deren Isomere sowie Additionssalze mit einer pharmazeutisch annehmbaren Säure oder Base.

7. Verbindungen der Formel (I) nach einem der Ansprüche 1 bis 6, **dadurch gekennzeichnet, daß** R eine Gruppe der Formel (ii) darstellt, wie sie bezüglich der Formel (I) definiert worden ist, in der:
◆ A eine Einfachbindung darstellt und
◆ G eine Gruppe der Formel -O-C(O)-NR₂-T₂ bedeutet, in der R₂ und T₂ die bezüglich der Formel (I) angegebenen Bedeutungen besitzen und vorzugsweise R₂ ein Wasserstoffatom oder eine geradkettige oder verzweigte (C₁-C₆)-Alkylgruppe darstellt und T₂ eine gegebenenfalls substituierte Arylgruppe oder eine geradkettige oder verzweigte (C₁-C₆)-Alkylenkette, die durch eine Gruppe -NR₃R₄ substituiert ist, in der R₃ und R₄ die bezüglich der Formel (I) angegebenen Bedeutungen besitzen, darstellt,
deren Isomere sowie deren Additionssalze mit einer pharmazeutisch annehmbaren Säure oder Base.

8. Verbindungen der Formel (I) nach einem der Ansprüche 1 bis 6, **dadurch gekennzeichnet, daß** R eine Gruppe der Formel (ii) darstellt, wie sie bezüglich der Formel (I) definiert worden ist, in der:
◆ A eine Einfachbindung darstellt und
◆ G eine Gruppe -NR₂-SO₂-T₃ bedeutet, in der R₂ und T₃ die bezüglich der Formel (I) angegebenen Bedeutungen besitzen, wobei vorzugsweise R₂ ein Wasserstoffatom oder eine geradkettige oder verzweigte (C₁-C₆)-Alkylgruppe darstellt und T₃ eine geradkettige oder verzweigte (C₁-C₆)-Alkylgruppe, eine Arylgruppe oder eine Heteroarylgruppe bedeutet,
deren Isomere sowie deren Additionssalze mit einer pharmazeutisch annehmbaren Säure oder Base.

9. Verbindungen der Formel (I) nach Anspruch 1, nämlich:
- 9-(4-Hydroxy-3,5-dimethoxyphenyl)-6-oxo-5,5a,6,8,8a,9-hexahydrofuro[3',4':6,7)naphtho[2,3-*d*][1,3]dioxol-5-yl-4-fluorphenylcarbamat,
- 9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-2-(dimethylamino)-ethyl(methyl)carbamat,
- 9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-2-(dimethylamino)-ethylcarbamat,
- 9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-3-(dimethylamino)-propyl(methyl)carbamat und
- 9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-3-(dimethylamino)-propylcarbamat,
deren Isomere sowie deren Additionssalze mit einer pharmazeutisch annehmbaren Säure oder Base.

10. Verbindungen der Formel (I) nach Anspruch 1, nämlich:
- (5R,5aR,8aS,9R)-9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-4-fluorphenylcarbamat,
- (5R,5aS,8aR,9R)-9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-2-(dimethylamino)-ethyl(methyl)carbamat,
- (5R,5aR,8aS,9R)-9-(4-Hydroxy-3,5-dimethoxyphenyl)-6-oxo-5,5a,6,8,8a,9-hexahydrofuro[3',4':6.7]naphtho[2,3-d][1,3]dioxol-5-yl-2-(dimethylamino)-ethylcarbamat,
- (5R,5aR,8aS,9R)-9-(4-Hydroxy-3,5-dimethoxyphenyl)-6-oxo-5,5a,6,8,8a,9-hexahydrofuro[3',4':6,7]naphtho[2,3-*d*)[1,3]dioxol-5-yl-3-(dimethylamino)-propyl(methyl)carbamat und
- (5R,5aS,8aR,9R)-9-(4-Hydroxy-3,5-dimethoxyphenyl)-6-oxo-5,5a,6,8,8a,9-hexahydrofuro[3',4':6,7]naphtho[2,3-*d*][1,3]dioxol-5-yl-3-(dimethylamino)-propylcarbamat.

11. Verfahren zur Herstellung der Verbindungen der Formel (I), **dadurch gekennzeichnet, daß** man als Ausgangsprodukt eine Verbindung der Formel (II) verwendet: 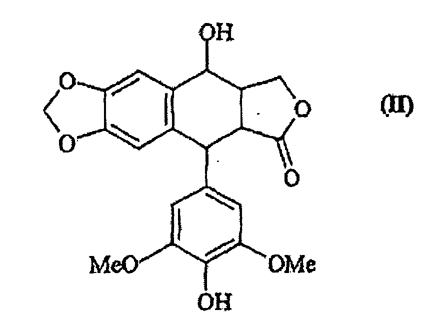 bei welcher Verbindung der Formel (II) die Hydroxyfunktionen unter Anwendung üblicher Verfahrensbedingungen der organischen Synthese mit einer klassischerweise in der organischen Chemie verwendeten Silylschutzgruppe geschützt werden zur Bildung der Verbindung der Formel (III): 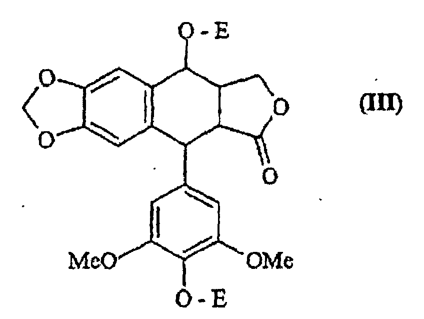 in der E eine Silylschutzgruppe bedeutet,
welche Verbindung der Formel (III) mit einem Reduktionsmittel behandelt wird zur Bildung der Verbindung der Formel (IV): 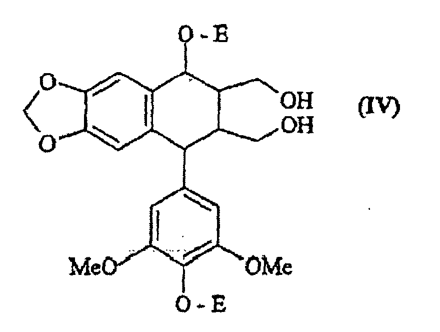 in der E die oben angegebene Bedeutung besitzt,
welche Verbindung der Formel (IV) in basischem Medium mit Tetrapropylammoniumperruthenat behandelt wird zur Bildung der Verbindungen der Formel (V): 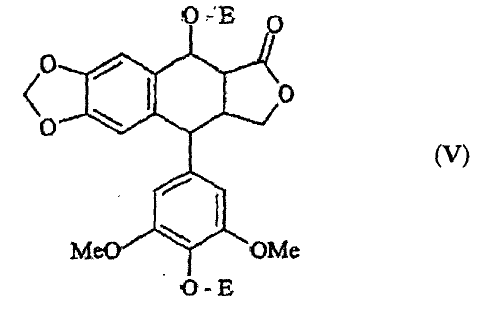 in der E die oben angegebene Bedeutung besitzt,
welche Verbindung der Formel (V) während mehrerer Stunden mit Tetrapropylammoniumfluorid behandelt wird zur Bildung der Verbindungen der Formel (I/a), einem Sonderfall der Verbindungen der Formel (1): 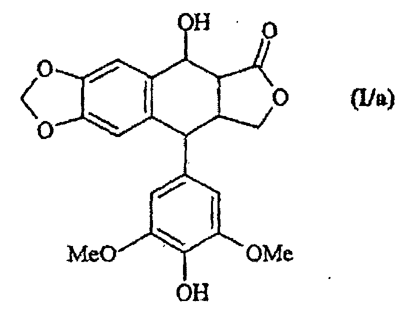 welche Verbindung der Formel (I/a) unter basischen Bedingungen der Einwirkung einer Verbindung der Formel (VII) unterworfen wird:
R'₁ - Y **(VII)**
in der R'₁ eine geradkettige oder verzweigte (C₁-C₆)-Alkylgruppe, Arylgruppe, geradkettige oder verzweigte Aryl-(C₁-C₆)-alkylgruppe, Heteroarylgruppe, geradkettige oder verzweigte Heteroaryl-(C₁-C₆)-alkylgruppe, geradkettige oder verzweigte (C₁-C₆)-Alkylcarbonylgruppe, Arylcarbonylgruppe, geradkettige oder verzweigte Aryl-(C₁-C₆)-alkylcarbonylgruppe, Heterocycloalkylcarbonylgruppe, geradkettige oder verzweigte (C₁-C₆)-Alkoxycarbonylgruppe, Aryloxycarbonylgruppe, geradkettige oder verzweigte Aryl-(C₁-C₆)-alkoxycarbonylgruppe, geradkettige oder verzweigte (C₁-C₆)-Alkylsulfonylgruppe, Arylsulfonylgruppe oder geradkettige oder verzweigte Aryl-(C₁-C₆)-alkylsulfonylgruppe und X ein Wasserstoffatom oder ein Halogenatom bedeuten, zur Bildung der Verbindungen der Formel (I/b), einem Sonderfall der Verbindungen der Formel (I): 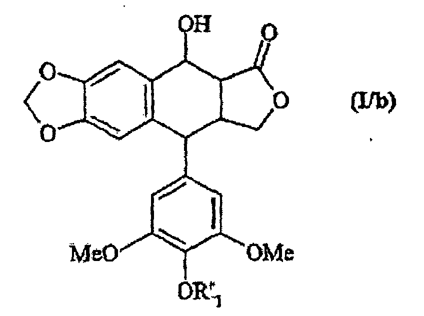 in der R'₁ die oben angegebenen Bedeutungen besitzt,
wobei die Gesamtheit der Verbindungen der Formeln (I/a) und (I/b) die Verbindungen der Formel (I/c) bilden: 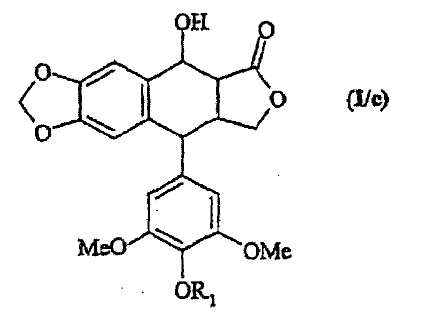 in der R₁ die bezüglich der Formel (I) angegebenen Bedeutungen besitzt,
welche Verbindung der Formel (I/c):
· **entweder mit einer Verbindung der Formel (VIII):** 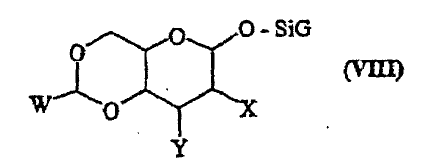 in der X, Y und W die bezüglich der Formel (I) angegebenen Bedeutungen besitzen und SiG eine in klassischer Weise in der Zuckerchemie verwendete Silylschutzgruppe bedeutet,
in Gegenwart von Bortrifluorid-etherat umgesetzt wird, zur Bildung der Verbindungen der Formel (I/d), einem Sonderfall der Verbindungen der Formel (I): 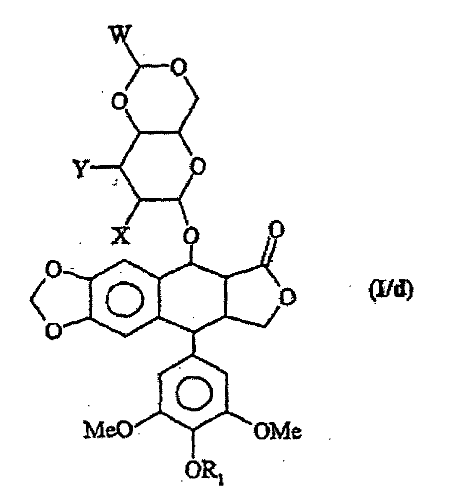 in der X, Y, W und R₁ die bezüglich der Formel (I) angegebenen Bedeutungen besitzen,
· **oder in Gegenwart einer Base mit einer Verbindung der Formel (IX):** 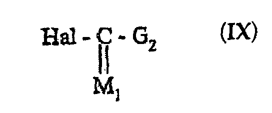 in der Hal ein Halogenatom, M₁ ein Sauerstoffatom oder ein Schwefelatom und G₂ eine Gruppe T₂ oder -O-T₂, worin T₂ die bezüglich der Formel (I) angegebenen Bedeutungen besitzt, bedeuten,
umgesetzt werden zur Bildung der Verbindungen der Formel (I/e), einem Sonderfall der Verbindungen der Formel (I): 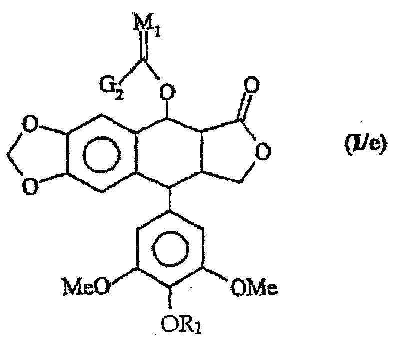 in der R₁ die bezüglich der Formel (I) angegebenen Bedeutungen besitzt und M₁ und G₂ die oben angegebenen Bedeutungen besitzen,
· **oder mit einer Verbindung der Formel (X):**
M₁ = C = N - T₂ **(X)**
in der M₁ die oben angegebenen Bedeutungen besitzt und T₂ die bezüglich der Formel (I) angegebenen Bedeutungen besitzt, umgesetzt wird
zur Bildung der Verbindungen der Formel (I/f), einem Sonderfall der Verbindungen der Formel (I): 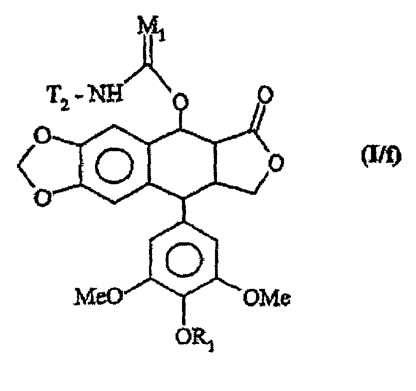 in der R₁, M₁ und T₂ die oben angegebenen Bedeutungen besitzen,
welche Verbindungen der Formel (I/f) der Einwirkung einer Verbindung der Formel (XI):
R'₂ - Hal **(XI)**
in der Hal ein Halogenatom bedeutet und R'₂ die Bedeutungen von R₂ besitzt, wie bezüglich der Formel (I) angegeben, mit Ausnahme der Bedeutung des Wasserstoffatoms, unterworfen werden können.
zur Bildung der Verbindungen der Formel (I/g), einem Sonderfall der Verbindungen der Formel (I): 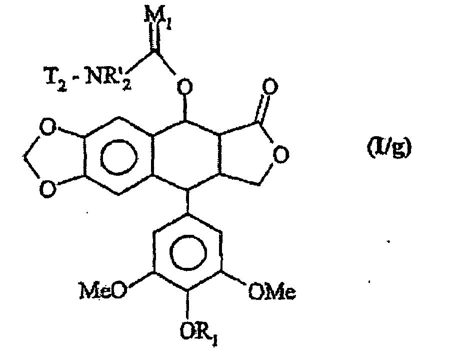 in der R₁, R'₂ T₂ und M₁ die oben angegebenen Bedeutungen besitzen,
· **oder mit Natriumazid umgesetzt werden zur Bildung der Verbindungen der Formel (XIa):** 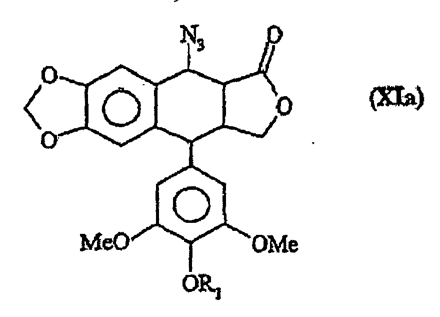 in der R₁ die oben angegebenen Bedeutungen besitzt,
bei welchen Verbindungen der Formel (XIa) man die Azidfunktion zu einer primären Aminfunktion reduziert und dann:
◆ entweder mit einer Verbindung der Formel (X), wie sie oben definiert worden ist, umsetzt zur Bildung der Verbindungen der Formel (I/h), einem Sonderfall der Verbindungen der Formel (I): 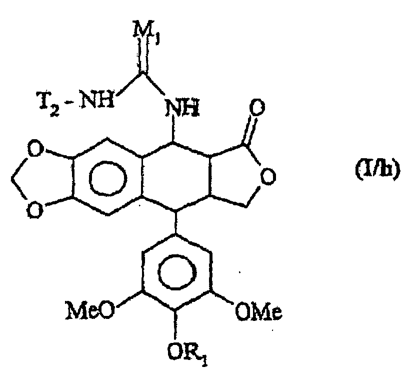 in der R₁, M₁ und T₂ die oben angegebenen Bedeutungen besitzen, bei welchen Verbindungen der Formel (I/h) die beiden Stickstoffatome der Harnstoff- oder Thioharnstoff-Funktion leicht unter klassischen Bedingungen der organischen Synthese durch Behandeln in basischem Medium mit einer Verbindung der Formel (XI) R'₂ - Hal, wie sie oben definiert worden ist, funktionalisiert werden können zur Bildung eines monofunktionalisierten oder bifunktionalisierten Harnstoffs oder Thioharnstoffs der Formel - NR₂-C(M₁)-NR₂-T₂.
◆ oder mit einer Verbindung der Formel (IX), wie sie oben definiert worden ist, umsetzt, zur Bildung der Verbindungen der Formel (I/i), einem Sonderfall der Verbindungen der Formel (I): 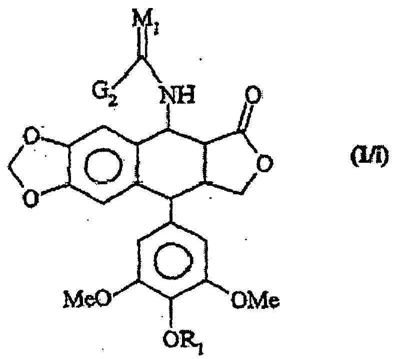 in der R₁, M₁ und G₂ die oben angegebenen Bedeutungen besitzen, bei welchen Verbindungen der Formel (I/i) das Stickstoffatom ohne weiteres unter klassischen Bedingungen der organischen Synthese durch Behandeln in basischem Medium mit einer Verbindung der Formel (XI), wie sie oben definiert worden ist, funktionalisiert werden kann,
◆ oder mit einer Verbindung der Formel (XII):
Cl - SO₂- T₃ **(XII)**
in der T₃ die gleichen Bedeutungen besitzt wie bezüglich der Formel (I) angegeben, umsetzt zur Bildung der Verbindungen der Formel (I/j), einem Sonderfall der Verbindungen der Formel (I): 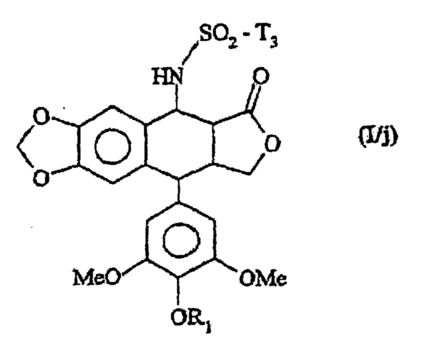 in der R₁ und T₃ die oben angegebenen Bedeutungen besitzen.
· **oder mit einer Verbindung der Formel (XIII):**
Hal - G₃ **(XIII)**
in der Hal ein Halogenatom bedeutet und G₃ eine Gruppe darstellt ausgewählt aus -R'₂, -T₁-NR₃R₄, -T₁-NR₂-T'₁-NR₃R₄, in denen R'₂, R₂, R₃, R₄, T₁ und T'₁ die oben angegebenen Bedeutungen besitzen, umgesetzt werden
zur Bildung der Verbindungen der Formel (I/k), einem Sonderfall der Verbindungen der Formel (I): 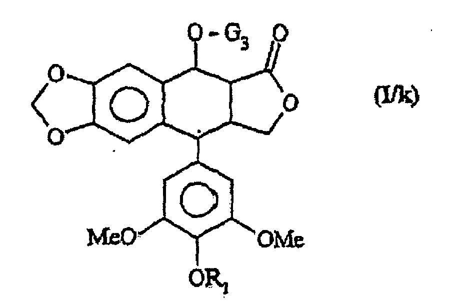 in der R₁ und G₃ die oben angegebenen Bedeutungen besitzen,
· **oder mit gasförmiger Chlorwasserstoffsäure oder Bromwasserstoffsäure umgesetzt werden zur Bildung der Verbindungen der Formel (XIV):** 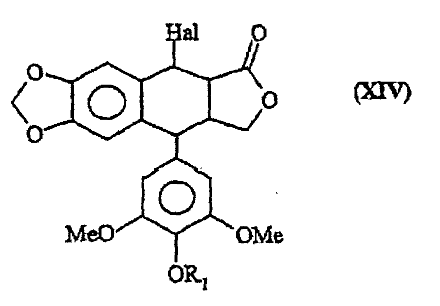 in der Hal ein Chlor- oder Bromatom bedeutet und R₁ die bezüglich der Formel (I) angegebenen Bedeutungen besitzt,
welche Verbindungen der Formel (XIV) man in basischem Medium mit einer Verbindung der Formel (XV) oder der Formel (XVbis):
H - N(R₂) - G₄ **(XV)** HN(R₄) - R₃ **(XVbis)**
in denen R₂, R₃ und R₄ die bezüglich der Formel (I) angegebenen Bedeutungen besitzen und G₄ eine Gruppe der Formel -T₁-NR₃R₄, -T₁-OR₅, -T₁-CO₂R₆ oder T₁-CO-R₆ darstellt, in denen T₁, R₃, R₄, R₅ und R₆ die bezüglich der Formel (I) angegebenen Bedeutungen besitzen, behandelt
zur Bildung der Verbindungen der Formel (I/1) und (I/m), einem Sonderfall der Verbindungen der Formel (I): 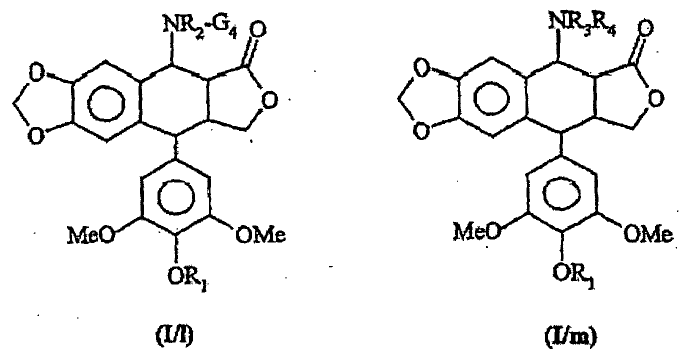 in denen R₁, R₂, R₃, R₄ und G₄ die oben angegebenen Bedeutungen besitzen,
· **oder in Gegenwart von Bortrifluorid-etherat mit einer Verbindung der Formel (XVI):**
Me₃Si - A₁ - CH = CH₂ **(XVI)**
in der A₁ eine geradkettige oder verzweigte (C₁-C₅)-Alkylenkette bedeutet, umgesetzt werden, zur Bildung der Verbindungen der Formel (I/n), einem Sonderfall der Verbindungen der Formel (I): 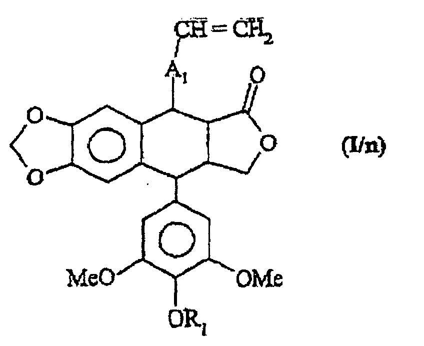 in der R₁ und A₁ die oben angegebenen Bedeutungen besitzen,
welche Verbindungen der Formel (I/n) man mit Osmiumtetraoxid in Gegenwart von N-Methylmorpholinoxid behandelt und dann durch Einwirkung von Bleitetraacetat oxidiert zur Bildung der Verbindungen der Formel (XVII): 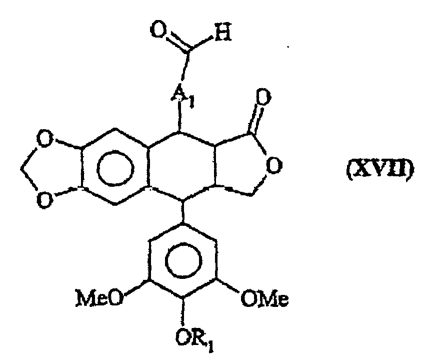 in der A₁ und R₁ die oben angegebenen Bedeutungen besitzen,
welche Verbindungen der Formel (XVII) anschließend ohne weiteres mit Hilfe klassischer Methoden der Reduktion, Oxidation und elektrophilen oder nucleophilen Addition, die dem auf dem Gebiet der organischen Synthese versierten Fachmann wohlbekannt sind, behandelt werden zur Bildung der Verbindungen der Formel (I/o), einem Sonderfall der Verbindungen der Formel (I): 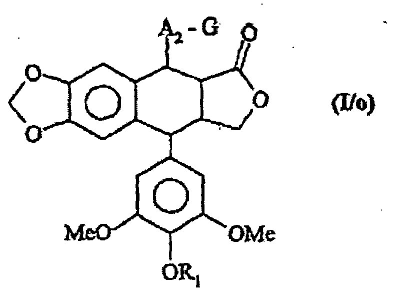 in der R₁ die oben angegebenen Bedeutungen besitzt, G die bezüglich der Formel (I) angegebenen Bedeutungen besitzt und A₂ die gleichen Bedeutungen besitzt wie die von A bezüglich der Formel (I), mit Ausnahme der Definition der Einfachbindung,
welche Verbindungen der Formeln (I/a) bis (I/o), welche die Gesamtheit der erfindungsgemäßen Verbindungen der Formel (I) bilden, man gegebenenfalls mit Hilfe einer klassischen Reinigungsmethode reinigt, gewünschtenfalls mit Hilfe klassischer Trennungsmethoden in ihre verschiedenen Isomeren aufgetrennt werden können und welche man gewünschtenfalls in ihre Additionssalze mit einer pharmazeutisch annehmbaren Säure oder Base umwandelt.

12. Verbindungen der Formeln (XIa), (XIV) und (XVII), wie sie in Anspruch 11 definiert sind, als Zwischenprodukte der Synthese für die Herstellung der Verbindungen der Formel (I).

13. Pharmazeutische Zubereitungen enthaltend als Wirkstoff mindestens eine Verbindung nach einem der Ansprüche 1 bis 10 allein oder in Kombination mit einem oder mehreren inerten, nichttoxischen, pharmazeutisch annehmbaren Trägermaterialien oder Bindemitteln.

14. Pharmazeutische Zubereitungen nach Anspruch 13 nützlich als Arzneimittel, enthaltend mindestens einen Wirkstoff nach einem der Ansprüche 1 bis 10 zur Behandlung von Krebs.

## Claims

1. Compounds of formula (I) : 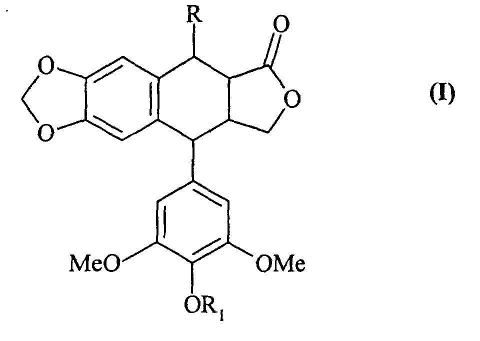 wherein :
R represents :
- *a group of formula (i) :* 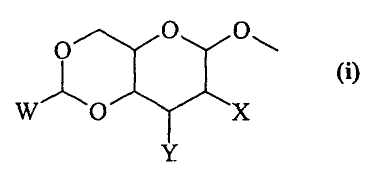 wherein :
X and Y, which may be identical or different, each represents a group selected from hydrogen, hydroxy, linear or branched (C₁₋C₆)alkoxy, amino, linear or branched (C₁-C₆)alkylamino and di-(C₁-C₆)alkylamino in which each alkyl moiety may be linear or branched,
W represents a hydrogen atom, a linear or branched (C₁-C₆)alkyl group, aryl or heteroaryl,
- *or a group of formula (ii):*
- A-G **(ii)**
wherein :
A represents a single bond or a linear or branched (C₁-C₆)alkylene chain optionally substituted by one or more identical or different groups selected from halogen and hydroxy and optionally containing an unsaturated bond,
G represents a group selected from hydrogen, cycloalkyl, heterocycloalkyl, aryl, heteroaryl, -OR₂, -O-T₁-NR₃R₄, -O-T₁-NR₂-T'₁NR₃R₄, -NR₃R₄, -NR₂-T₁-NR₃R₄, - NR₂-T₁-OR₅, -NR₂-T₁-CO₂R₆, -NR₂-T₁-C(O)R₆, -C(O)-NR₃R₄, -C(O)-NR₂-T₂, -O-C(O)-T₂, -O-C(S)-T₂, -NR₂-C(O)-T₂, -NR₂-C(S)-T₂, -O-C(O)-O-T₂, -O-C(O)-NR₂-T₂, -O-C(S)-O-T₂, -O-C(S)-NR₂-T₂, NR₂-C(O)-O-T₂, -NR₂-C(O)NR₆-T₂, -NR₂-C(S)-O-T₂, -NR₂-C(S)-NR₆-T₂ and -NR₂-SO₂-T₃ wherein :
R₂ represents a hydrogen atom, a linear or branched (C₁-C₆)alkyl group, aryl or aryl-(C₁-C₆)alkyl in which the alkyl moiety may be linear or branched,
T₁ and T'₁, which may be identical or different, each represents a linear or branched (C₁-C₆)alkylene chain,
R₃ and R₄, which may be identical or different, each represents independently of the other:
- a hydrogen atom, a linear or branched (C₁-C₆)alkyl group (optionally substituted by one or more hydroxy groups), aryl, aryl-(C₁-C₆)alkyl in which the alkyl moiety may be linear or branched, cycloalkyl, heteroaryl-(C₁-C₆)alkyl in which the alkyl moiety may be linear or branched, heteroaryl, heterocycloalkyl or heterocycloalkyl-(C₁-C₆)alkyl in which the alkyl moiety may be linear or branched,
- or form together with the nitrogen atom carrying them a saturated or unsaturated, 5- to 7-membered, monocyclic heterocycle optionally containing a second hetero atom selected from oxygen and nitrogen, the said heterocycle being optionally substituted by one or more groups selected from halogen, hydroxy, linear or branched (C₁-C₆)alkyl and heterocycles,
R₅ represents a linear or branched (C₁-C₆)alkyl group, aryl or aryl-(C₁-C₆)alkyl in which the alkyl moiety may be linear or branched,
R₆ represents a hydrogen atom, a linear or branched (C₁-C₆)alkyl group, aryl or aryl-(C₁-C₆)alkyl in which the alkyl moiety may be linear or branched,
T₂ represents a group selected from hydrogen, linear or branched (C₁-C₆)alkyl (optionally substituted by one or more halogen atoms), aryl, aryl-(C₁-C₆)alkyl in which the alkyl moiety may be linear or branched, cycloalkyl, cycloalkyl-(C₁-C₆)alkyl in which the alkyl moiety may be linear or branched, heterocycloalkyl and heterocycloalkyl-(C₁-C₆)alkyl in which the alkyl moiety may be linear or branched, or T₂ represents a linear or branched (C₁-C₆)alkylene chain, the said chain being substituted by one or more identical or different groups selected from -NR₃R₄, -OR₂, -CO₂R₆, -NR₂-C(O)R₆, -NR₂-CO₂R₆ -C(O)R₆, -C(O)NR₃R₄, - NR₂-T₁-NR₃R₄, -NR₂-T₁-OR₆ and -O-T₁-NR₃R₄ wherein R₂, R₃, R₄, R₆ and T₁ are as defined hereinbefore,
T₃ represents a group selected from linear or branched (C₁-C₂₀)alkyl (optionally substituted by one or more groups selected from halogen, -OR₂, -NR₂R₆, nitro, cyano and azide), aryl, aryl-(C₁-C₆)alkyl in which the alkyl moiety may be linear or branched, cycloalkyl, cycloalkyl-(C₁-C₆)alkyl in which the alkyl moiety may be linear or branched, heterocycloalkyl, heterocycloalkyl-(C₁-C₆)alkyl in which the alkyl moiety may be linear or branched, heteroaryl and heteroaryl-(C₁-C₆)alkyl in which the alkyl moiety may be linear or branched,
R₁ represents a group selected from hydrogen, linear or branched (C₁-C₆)alkyl, aryl, aryl-(C₁-C₆)alkyl in which the alkyl moiety may be linear or branched, heteroaryl, heteroaryl-(C₁-C₆)alkyl in which the alkyl moiety may be linear or branched, linear or branched (C₁-C₆)alkylcarbonyl, arylcarbonyl, aryl-(C₁-C₆)alkylcarbonyl in which the alkyl moiety may be linear or branched, heterocycloalkylcarbonyl, linear or branched (C₁-C₆)alkylsulphonyl, arylsulphonyl, aryl-(C₁-C₆)alkylsulphonyl in which the alkyl moiety may be linear or branched, phosphonic groups, linear or branched (C₁-C₆)alkoxycarbonyl, aryloxycarbonyl and aryl-(C₁-C₆)alkoxycarbonyl in which the alkoxy moiety may be linear or branched,
their isomers, and also addition salts thereof with a pharmaceutically acceptable acid or base,
wherein:
* *aryl* denotes a phenyl, biphenyl, naphthyl, dihydronaphthyl, tetrahydronaphthyl, indenyl or indanyl group, each of those groups optionally having one or more identical or different substituents selected from halogen, hydroxy, linear or branched (C₁-C₆)alkyl, linear or branched (C₁-C₆)alkoxy, cyano, nitro, amino, linear or branched (C₁-C₆)alkylamino, di-(C₁-C₆)alkylamino in which each alkyl moiety may be linear or branched, linear or branched (C₁-C₆)alkylsulphonyl, linear or branched (C₁-C₆)alkylsulphonylamino, carboxy, linear or branched (C₁-C₆)alkoxycarbonyl, aryloxycarbonyl, aryl(C₁-C₆)alkoxycarbonyl in which the alkoxy moiety may be linear or branched, linear or branched (C₁-C₆)hydroxyalkyl, linear or branched (C₁-C₆)trihaloalkyl, methylenedioxy, ethylenedioxy, morpholinyl, piperidyl, piperazinyl, linear or branched (C₁-C₆)alkylcarbonyloxy and linear or branched (C₁-C₆)alkylcarbonyl,
* *heteroaryl* denotes an aromatic monocyclic group, an aromatic bicyclic group, or a bicyclic group in which one of the rings is aromatic and the other ring is partially hydrogenated, having from 5 to 12 ring members and containing in the ring system one, two or three identical or different hetero atoms selected from oxygen, nitrogen and sulphur, it being possible for the said heteroaryl optionally to be substituted by the same substituents as those decribed in the case of the aryl group,
* *cycloalkyl* denotes a monocyclic or bicyclic group that is saturated or unsaturated, but not of aromatic character, that contains from 3 to 10 carbon atoms and is optionally substituted by one or more identical or different groups selected from halogen, linear or branched (C₁-C₆)alkyl, linear or branched (C₁-C₆)trihaloalkyl, hydroxy, linear or branched (C₁-C₆)hydroxyalkyl, amino, linear or branched (C₁-C₆)alkylamino, di-(C₁-C₆)-alkylamino in which each alkyl moiety may be linear or branched, piperidyl, piperazinyl and morpholinyl,
* *heterocycloalkyl* is to be understood as a cycloalkyl group as defined above containing one or two identical or different hetero atoms selected from oxygen, nitrogen and sulphur, the said heterocycloalkyl being optionally substituted by one or more substituents such as those described in the case of the cycloalkyl group,
with the proviso that R₁ does not represent a methyl group when R represents a group -A-G in which A represents a single bond and G represents a hydrogen atom.

2. Compounds of formula (I) according to claim 1, **characterised in that** R₁ represents a group selected from hydrogen, linear or branched (C₁-C₆)alkyl, linear or branched (C₁-C₆)-alkoxycarbonyl, aryloxycarbonyl and aryl-(C₁-C₆)alkoxycarbonyl in which the alkoxy moiety may be linear or branched, their isomers, and also addition salts thereof with a pharmaceutically acceptable acid or base.

3. Compounds of formula (I) according to either claim 1 or claim 2, **characterised in that** R₁ represents a hydrogen atom, their isomers, and also addition salts thereof with a pharmaceutically acceptable acid or base.

4. Compounds of formula (I) according to claim 1, **characterised in that** R represents a group of formula (i) as defined for formula (I), wherein :
◆ X represents an amino group, linear or branched (C₁-C₆)alkylamino or di-(C₁-C₆)alkylamino in which each alkyl moiety may be linear or branched, and Y represents a hydroxy group; or X and Y are identical and each represents a hydroxy group; or X represents a hydrogen atom and Y represents an amino group, linear or branched (C₁-C₆)alkylamino or di-(C₁-C₆)alkylamino in which each alkyl moiety may be linear or branched and
◆ W represents a methyl or 2-thienyl group,
their isomers, and also addition salts thereof with a pharmaceutically acceptable acid or base.

5. Compounds of formula (I) according to either claim 1 or claim 4, **characterised in that** R represents a group of formula (i) such as: 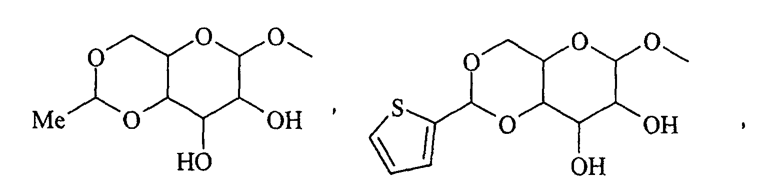 or 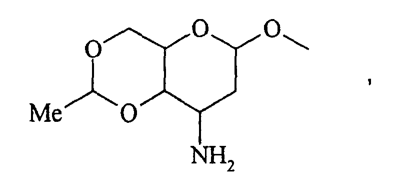 their isomers, and also addition salts thereof with a pharmaceutically acceptable acid or base.

6. Compounds of formula (I) according to claim 1, **characterised in that** R represents a group of formula (ii) as defined for formula (1) wherein:
◆ A represents a single bond and
◆ G represents a group selected from O-T₁-NR₃R₄, -O-T₁-NR₂-T'₁-NR₃R₄, - NR₂-T₁-NR₃R₄, -NR₃R₄, -NR₂-T₁-OR₅, -O-C(O)-NR₂-T₂ and -NR₂-SO₂-T₃ in which T₁, T'₁, T₂, T₃, R₂, R₃, R₄ et R₅ are as defined for formula (I),
their isomers, and also addition salts thereof with a pharmaceutically acceptable acid or base.

7. Compounds of formula (I) according to either claim 1 or claim 6, **characterised in that** R represents a group of formula (ii) as defined for formula (I), wherein:
◆ A represents a single bond and
◆ G represents a group -O-C(O)-NR₂-T₂ wherein R₂ and T₂ are as defined for formula (I) and, advantageously, R₂ represents a hydrogen atom or a linear or branched (C₁-C₆)alkyl group and T₂ represents an optionally substituted aryl group or a linear or branched (C₁-C₆)alkylene chain substituted by an -NR₃R₄ group wherein R₃ and R₄ are as defined for formula (I),
their isomers, and also addition salts thereof with a pharmaceutically acceptable acid or base.

8. Compounds of formula (I) according to either claim 1 or claim 6, **characterised in that** R represents a group of formula (ii) as defined for formula (1) wherein:
◆ A represents a single bond and
◆ G represents a group -NR₂-SO₂-T₃ wherein R₂ and T₃ are as defined for formula (I) and, advantageously, R₂ represents a hydrogen atom or a linear or branched (C₁-C₆)alkyl group and T₃ represents a linear or branched (C₁-C₆)alkyl group, aryl or heteroaryl,
their isomers, and also addition salts thereof with a pharmaceutically acceptable acid or base.

9. Compounds of formula (I) according to claim 1 that are :
- 9-(4-hydroxy-3,5-dimethoxyphenyl)-6-oxo-5,5a,6,8,8a,9-hexahydrofuro[3',4':6,7]-naphtho[2,3-*d*][1,3]dioxol-5-yl 4-fluorophenylcarbamate,
- 9-(4-hydroxy-3,5-dimethoxyphenyl)-6-oxo-5,5a,6,8,8a,9-hexahydrofuro[3',4':6,7)-naphtho[2,3-*d*][1,3]dioxol-5-yl 2-(dimethylamino)ethyl(methyl)carbamate,
- 9-(4-hydroxy-3,5-dimethoxyphenyl)-6-oxo-5,5a,6,8,8a,9-hexahydrofuro[3',4':6,7]-naphtho[2,3-*d*][1,3]-dioxol-5-yl 2-(dimethylamino)ethylcarbamate,
- 9-(4-hydroxy-3,5-dimethoxyphenyl)-6-oxo-5,5a,6,8,8a,9-hexahydrofuro[3',4':6,7]-naphtho[2,3-*d*][1,3]dioxol-5-yl 3-(dimethylamino)propyl(methyl)carbamate,
- and 9-(4-hydroxy-3,5-dimethoxyphenyl)-6-oxo-5,5a,6,8,8a,9-hexahydrofuro[3',4':6,7]-naphtho[2,3-*d*][1,3]dioxol-5-yl 3-(dimethylamino)propylcarbamate,
their isomers, and also addition salts thereof with a pharmaceutically acceptable acid or base.

10. Compounds of formula (I) according to claim 1 that are:
- (5*R*,5a*R*,8a*S*,*9R*)-9-(4-hydroxy-3,5-dimethoxyphenyl)-6-oxo-5,5a,6,8,8a,9-hexahydrofuro[3',4':6,7]naphtho[2,3-*d*][1,3]dioxol-5-yl 4-fluorophenylcarbamate,
- (5*R*,5a*S*,8a*R*,9*R*)-9-(4-hydroxy-3,5-dimethoxyphenyl)-6-oxo-5,5a,6,8,8a,9-hexahydrofuro[3',4':6,7]naphtho[2,3-*d*][1,3]dioxol-5-yl 2-(dimethylamino)ethyl(methyl)-carbamate,
- (5*R*,5a*R*,8a*S*,9*R*)-9-(4-hydroxy-3,5-dimethoxyphenyl)-6-oxo-5,5a,6,8,8a,9-hexahydrofuro[3',4':6,7]naphtho[2,3-*d*][1,3]-dioxol-5-yl 2-(dimethylamino)ethylcarbamate,
- (5*R*,5a*R*,8a*S*,9*R*)-9-(4-hydroxy-3,5-dimethoxyphenyl)-6-oxo-5,5a,6,8,8a,9-hexahydrofuro[3',4':6,7]naphtho[2,3-*d*][1,3]dioxol-5-yl 3-(dimethylamino)propyl(methyl)carbamate and
- (5R,5aS,8aR,9R)-9-(4-hydroxy-3,5-dimethoxyphenyl)-6-oxo-5,5a,6,8,8a,9-hexahydrofuro[3',4':6,7]naphtho[2,3-*d*][1,3]dioxol-5-yl 3-(dimethylamino)propylcarbamate.

11. Process for the preparation of the compounds of formula (I), **characterised in that** there is used as starting material a compound of formula (II): 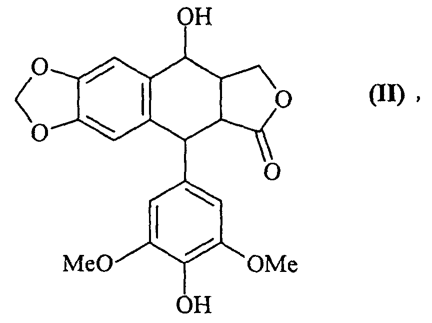 the hydroxy functions of which compound of formula (II) are protected by a silyl protecting group conventionally used in organic chemistry, in accordance with the operating conditions customary in organic synthesis, to yield a compound of formula (III) : 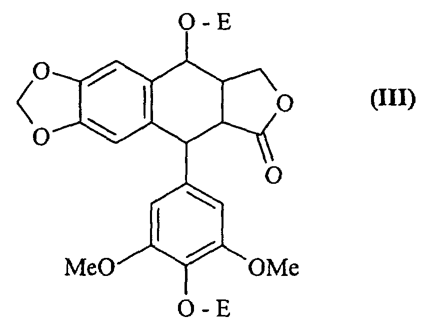 wherein E represents a silyl protecting group,
which compound of formula (III) is treated with a reducing agent to yield a compound of formula (IV) : 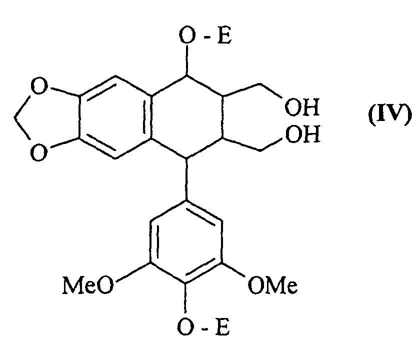 wherein E is as defined hereinbefore,
which compound of formula (IV) is reacted in basic medium, in the presence of tetrapropylammonium perruthenate, to yield the compounds of formula (V) : 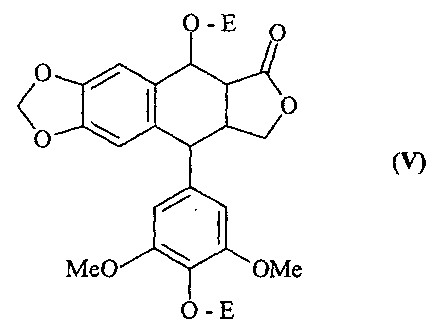 wherein E is as defined hereinbefore,
which compound of formula (V) is treated for several hours with tetrabutylammonium fluoride to yield the compounds of formula (I/a), a particular case of the compounds of formula (I) : 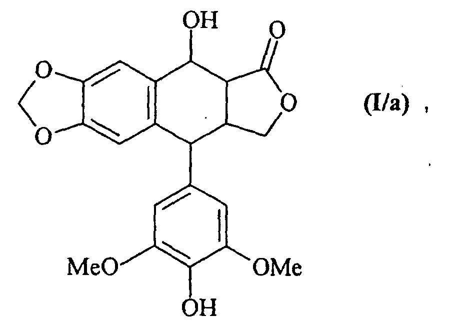 which compound of formula (I/a) is subjected, under basic conditions, to the action of a compound of formula (VII) :
R'₁ - X **(VII)**
wherein R'₁, represents a linear or branched (C₁-C₆)alkyl group, aryl, aryl-(C₁-C₆)alkyl in which the alkyl moiety may be linear or branched, heteroaryl, heteroaryl-(C₁-C₆)alkyl in which the alkyl moiety may be linear or branched, linear or branched (C₁-C₆)alkylcarbonyl, arylcarbonyl, aryl-(C₁-C₆)alkylcarbonyl in which the alkyl moiety may be linear or branched, heterocycloalkylcarbonyl, linear or branched (C₁-C₆)alkoxycarbonyl, aryloxycarbonyl, aryl-(C₁-C₆)alkoxycarbonyl in which the alkoxy moiety may be linear or branched, linear or branched (C₁-C₆)alkylsulphonyl, arylsulphonyl or aryl-(C₁-C₆)alkylsulphonyl in which the alkyl moiety may be linear or branched, and X represents a hydrogen atom or a halogen atom, to yield the compounds of formula (I/b), a particular case of the compounds of formula (1) : 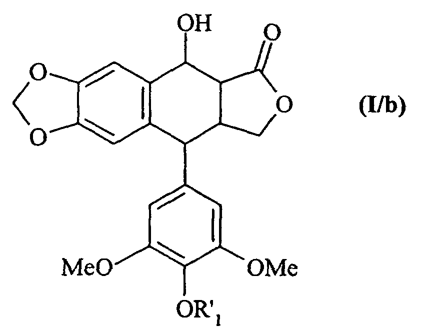 wherein R'₁ is as defined hereinbefore,
the totality of the compounds of formulae (I/a) and (I/b) constituting the compounds of formula (I/c): 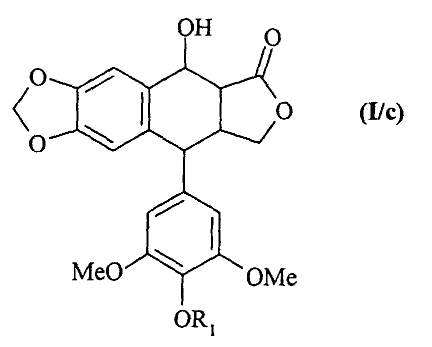 wherein R₁ is as defined for formula (I),
which compound of formula (I/c) is reacted :
• **with a compound of formula (VIII):** 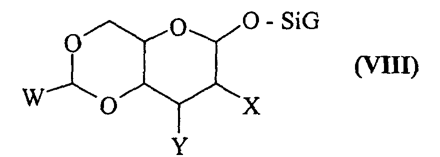 wherein X, Y et W are as defined for formula (I) and SiG represents a silyl protecting group conventionally used in sugar chemistry,
in the presence of boron trifluoride etherate, to yield the compounds of formula (I/d), a particular case of the compounds of formula (I): 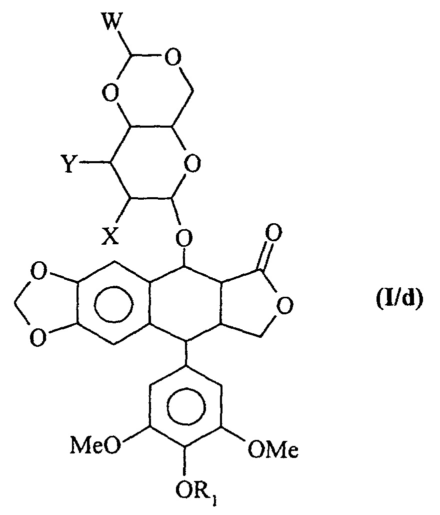 wherein X, Y, Wand R₁ are as defined for formula (I),
• or, in the presence of a base, with a compound of formula (IX): 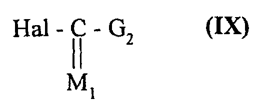 wherein Hal represents a halogen atom, M₁ represents an oxygen or sulphur atom and G₂ represents a group T₂ or -O-T₂ wherein T₂ is as defined for formula (I),
to yield the compounds of formula (I/e), a particular case of the compounds of formula (1) : 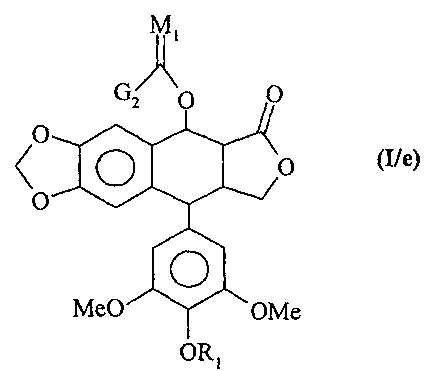 wherein R₁ is as defined for formula (I) and M₁ and G₂ are as defined hereinbefore,
• **or with a compound of formula (X) :**
M₁ = C = N - T₂ **(X)**
wherein M₁ is as defined hereinbefore and T₂ is as defined for formula (I),
to yield the compounds of formula (I/f), a particular case of the compounds of formula (I) : 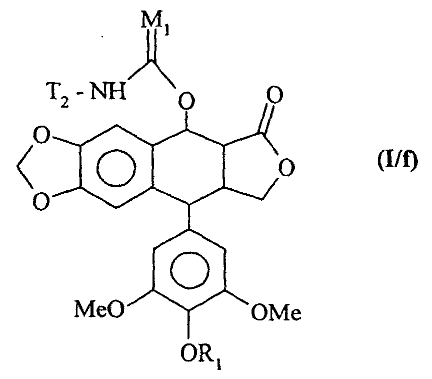 wherein R₁, M₁ and T₂ are as defined hereinbefore,
which compounds of formula (I/f) may be subjected to the action of a compound of formula (XI):
R'₂ - Hal **(XI)**
wherein Hal represents a halogen atom and R'₂ has the same meanings as R₂ in formula (I) with the exception of a hydrogen atom,
to yield the compounds of formula (I/g), a particular case of the compounds of formula (I): 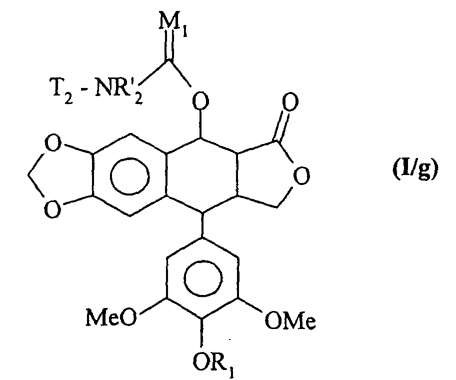 wherein R₁, R'₂, T₂ and M₁ are as defined hereinbefore,
• **or with sodium azide to yield the compounds of formula (XIa) :** 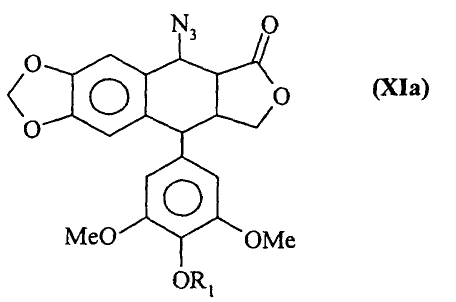 wherein R₁ is as defined hereinbefore,
the azide function of which compounds of formula (XIa) is reduced to the primary amine function, and which compounds are then reacted :
◆ either with a compound of formula (X) as defined hereinbefore to yield the compounds of formula (1/h), a particular case of the compounds of formula (I) : 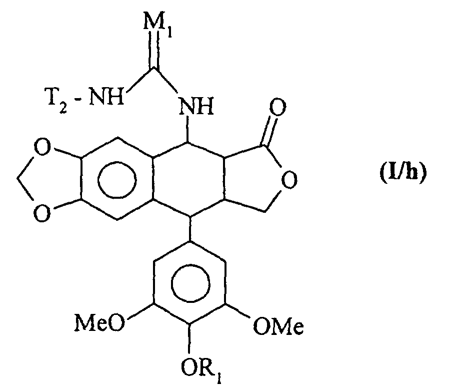 wherein R₁, M₁ and T₂ are as defined hereinbefore, the two nitrogen atoms of the urea or thiourea function of which compounds of formula (I/h) can readily be functionalised, in accordance with the conditions conventional in organic synthesis, by treatment in basic medium with a compound of formula (XI) R'₂ - Hal as defined hereinbefore, to yield a monofunctionalised or difunctionalised urea or thiourea of formula -NR₂-C(M₁)-NR₂-T₂,
◆ or with a compound of formula (IX) as defined hereinbefore to yield the compounds of formula (I/i), a particular case of the compounds of formula (I): 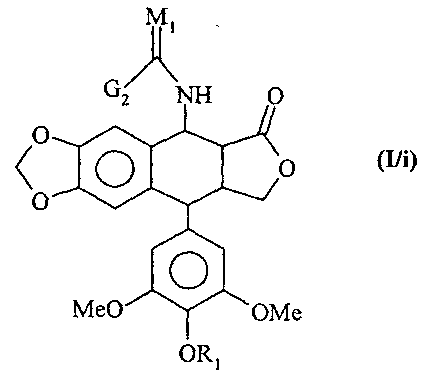 wherein R₁, M₁ and G₂ are as defined hereinbefore, the nitrogen atom of which compounds of formula (I/i) can readily be functionalised, in accordance with the conditions conventional in organic synthesis, by treatment in basic medium with a compound of formula (XI) as defined hereinbefore,
◆ or with a compound of formula (XII) :
Cl - SO₂ - T₃ **(XII)**
wherein T₃ has the same definition as for formula (I) to yield the compounds of formula (I/j), a particular case of the compounds of formula (I): 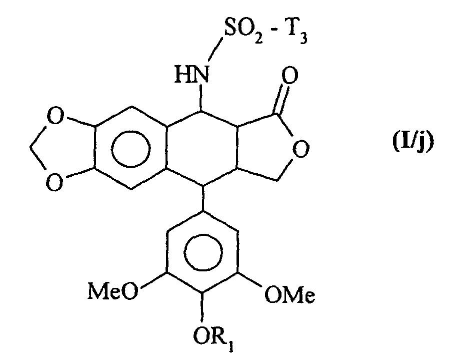 wherein R₁ and T₃ are as defined hereinbefore,
• **or with a compound of formula (XIII) :**
Hal - G₃ **(XIII)**
wherein Hal represents a halogen atom and G₃ represents a group selected from -R'₂, - T₁-NR₃R₄ and -T₁-NR₂-T'₁-NR₃R₄ wherein R'₂, R₂, R₃, R₄, T₁ and T'₁, are as defined hereinbefore,
to yield the compounds of formula (I/k), a particular case of the compounds of formula (I) : 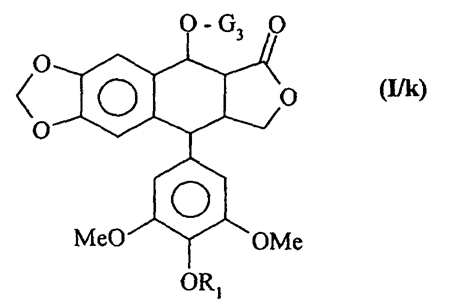 wherein R₁ and G₃ are as defined hereinbefore,
• **or with gaseous hydrogen chloride or hydrogen bromide to yield the compounds of formula (XIV):** 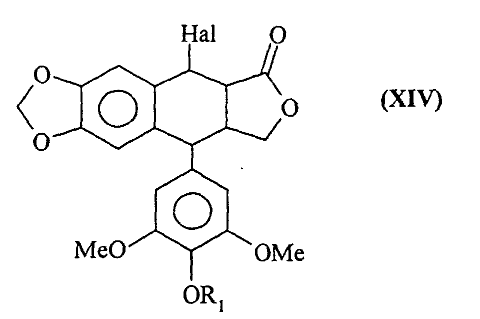 wherein Hal represents a chlorine or bromine atom and R₁ is as defined for formula (I), which compounds of formula (XIV) are treated in basic medium with a compound of formula (XV) or of formula (XVbis) :
H - N(R₂) - G₄ **(XV)** HN(R₄) - R₃ **(XVbis)**,
in which formulae R₂, R₃ and R₄ are as defined for formula (I) and G₄ represents a group of formula -T₁-NR₃R₄, -T₁-OR₅, -T₁-CO₂R₆ or T₁-CO-R₆ wherein T₁, R₃, R₄, R₅ and R₆ have the same meanings as for formula (I),
to yield the compounds of formula (I/1) and (I/m), a particular case of the compounds of formula (I), 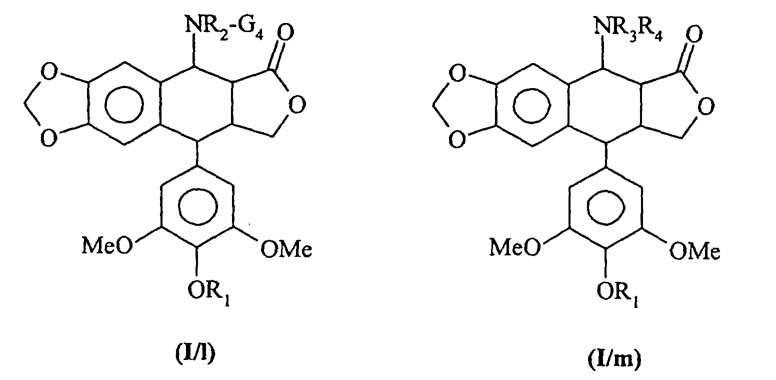 wherein R₁, R₂, R₃, R₄ and G₄ are as defined hereinbefore,
• **or is reacted, in the presence of boron trifluoride etherate, with a compound of formula (XVI) :**
Me₃Si - A₁ - CH = CH₂ **(XVI)**
wherein A₁ represents a linear or branched (C₁-C₅)alkylene chain to yield the compounds of formula (I/n), a particular case of the compounds of formula (1) : 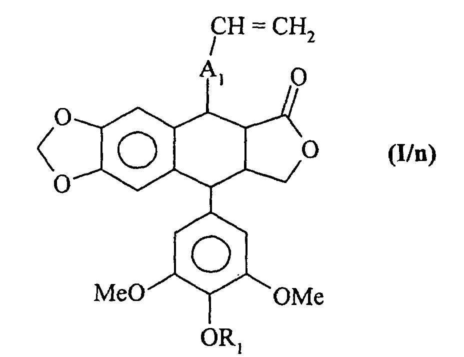 wherein R₁ and A₁ are as defined hereinbefore,
which compounds of formula (I/n) are treated with osmium tetroxide in the presence of N-methylmorpholine oxide and then oxidised by the action of lead tetraacetate to yield the compounds of formula (XVII): 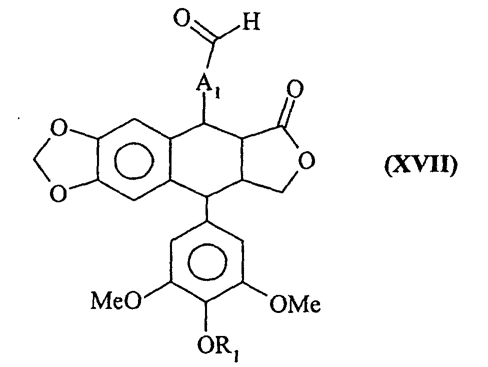 wherein A₁ and R₁ are as defined hereinbefore,
which compounds of formula (XVII) are then easily treated, by conventional methods of reduction, oxidation or electrophilic or nucleophilic addition that are well known to the person skilled in the art of organic synthesis, to yield the compounds of formula (I/o), a particular case of the compounds of formula (I): 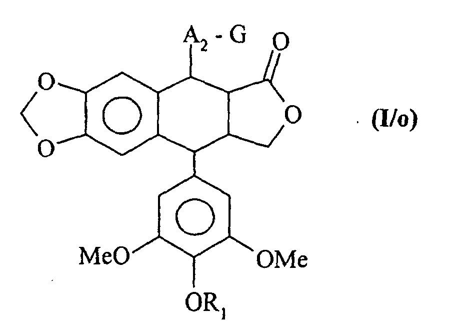 wherein R₁ is as defined hereinbefore, G has the same definition as for formula (I) and A₂ has the same meanings as A in formula (I) with the exception of the definition single bond,
the compounds (I/a) to (I/o) constituting the totality of the compounds of formula (I) of the invention, which compounds are purified, if necessary, according to a conventional purification technique, may be separated, if desired, into their different isomers according to a conventional separation technique, and are optionally converted into their addition salts with a pharmaceutically acceptable acid or base.

12. Compounds of formula (XIa), (XIV) and (XVII) as described in claim 11, for use as synthesis intermediates for the preparation of the compounds of formula (I).

13. Pharmaceutical compositions comprising as active ingredient at least one compound according to any one of claims 1 to 10, alone or in combination with one or more pharmaceutically acceptable, inert, non-toxic excipients or carriers.

14. Pharmaceutical compositions according to claim 13, for use as medicaments, comprising at least one active ingredient according to any one of claims 1 to 10, for use in the treatment of cancer.
